# EUROPEAN PATENT APPLICATION

(11) **EP 4 560 317 A1**
(43) Date of publication of application: **28.05.2025**
(21) Application number: 24208060.4
(22) Date of filing: 22.10.2024
(51) Int. Cl.: G01N 33/569, G01N 33/574

(54) **METHOD FOR DETECTING CIRCULATING TUMOR CELLS IN BLOOD**

(30) Priority: 22.11.2023 JP 2023198480
(71) Applicant: Osaka Prefectural Hospital Organization, Chuo-ku Osaka-shi, Osaka 541-8567 (JP); SYSMEX CORPORATION, Kobe-shi Hyogo 651-0073 (JP)
(72) Inventor: MIYOSHI, Norikatsu, Osaka-shi, Osaka, 541-8567 (JP); FUJINO, Shiki, Osaka-shi, Osaka, 541-8567 (JP); IJIRI, Yuichi, Kobe-shi, Hyogo, 651-0073 (JP); TAKAHASHI, Yusuke, Kobe-shi, Hyogo, 651-0073 (JP); YANAGIDA, Masatoshi, Kobe-shi, Hyogo, 651-0073 (JP)
(74) Representative: Hoffmann Eitle

(57) **Abstract**

Disclosed is a method for detecting circulating tumor cells containing defective APC protein, comprising immunostaining circulating tumor cells in the blood using a labeled antibody that binds to both wild-type and defective APC proteins, and a labeled antibody that binds to wild-type but not defective APC proteins, and measuring the signal derived from the label.

## Description

### [Technical field]

The present invention relates to a method for detecting circulating tumor cells (hereinafter also referred to as "CTCs").

### [Background Art]

Cancer cells often harbor mutations, such as nonsense mutations and frameshift mutations, in genes that code for specific proteins. In the transcript from a gene having such a mutation, a stop codon may be present at a position where protein synthesis is interrupted, or an amino acid sequence different from the original amino acid sequence may be encoded from the middle of the sequence. In such cases, cancer cells express proteins that are missing parts of their original form. For example, Non-Patent Document 1 describes that the colon cancer cell line DLD-1, which expresses only a defective form of the APC (adenomatous polyposis coli) protein, was not detected by immunostaining using an antibody that binds to the C-terminus of APC.

### [Prior art references]

### [Non-patent literature]

[Non-Patent Document 1] Neufeld KL and White RL, Nuclear and cytoplasmic localizations of adenomatous polyposis coli protein, Proc. Natl. Acad. Sci. USA, vol. 94, pp. 3034-3039, April 1997

### Summary of the Invention

### [Problem to be solved by the invention]

Defective proteins in cancer cells are thought to be related to tumor formation, recurrence, metastasis, and the like. In addition, some cancer cells are detached from tumor tissue, infiltrate into blood vessels, and circulate in the blood. Such cancer cells are called CTCs and are thought to be one of the causes of cancer recurrence and metastasis. Because CTCs are also cancer cells, they may contain defective proteins characteristic of the cancer type from which they originate, such as defective APC protein in colon cancer cells. Therefore, detecting CTCs that have defective proteins is clinically useful. An object of the present invention is to provide a method for detecting CTCs containing a defective APC protein.

### [Means for solving the problem]

The present invention provides the following inventions [1] to [15].
[1] A method for detecting a circulating tumor cell in a sample, comprising the steps of: contacting the circulating tumor cell in the sample with a first labeled antibody comprising a first labeling substance and a second labeled antibody comprising a second labeling substance that is different from the first labeling substance; and detecting a circulating tumor cell containing a defective APC protein to which the first labeled antibody is bound and to which the second labeled antibody is not bound, wherein the first labeled antibody comprises an antibody capable of binding to a wild-type APC protein and a defective APC protein, and the second labeled antibody comprises an antibody capable of binding to the wild-type APC protein and not to the defective APC protein.
[2] The detection method described in [1] above, further comprising the step of detecting cells containing a wild-type APC protein bound to the first labeled antibody and the second labeled antibody.
[3] The detection method according to [1] or [2] above, wherein the detecting step comprises: detecting a first signal derived from the first labeling substance and a second signal derived from the second labeling substance; and detecting a circulating tumor cell containing the defective APC protein based on the first signal and the second signal.
[4] The detection method according to any one of [1] to [3] above, wherein the detecting step comprises: detecting a first signal derived from the first labeling substance and a second signal derived from the second labeling substance; and detecting a cell in which a ratio of the second signal to the first signal is less than a threshold value, or a cell in which a ratio of the first signal to the second signal is equal to or greater than a threshold value, as a circulating tumor cell comprising the defective APC protein.
[5] The detection method according to any one of [1] to [4] above, wherein the detecting step comprises: acquiring an image of a cell in the sample, and detecting a first signal derived from the first labeling substance and a second signal derived from the second labeling substance in the image of the cell; and detecting a cell comprising the first signal and substantially not comprising the second signal as a circulating tumor cell containing the defective APC protein.
[6] The detection method described in [5] above, wherein the first labeling substance is a fluorescent substance, the second labeling substance is a fluorescent substance, the first signal is a fluorescent signal, the second signal is a fluorescent signal, and the image is a fluorescent image.
[7] The detection method according to any one of [1] to [6] above, wherein the defective APC protein is a protein in which the C-terminal region of the wild-type APC protein is deleted.
[8] The detection method according to any one of the above [1] to [7], wherein the first labeling substance and the second labeling substance are fluorescent substances having fluorescence emission maxima in different wavelength ranges.
[9] The detection method according to any one of the above-mentioned [1] to [8], further comprising the steps of: labeling a mesenchymal marker protein of the circulating tumor cell; and detecting a cell to which the first labeled antibody binds, in which the mesenchymal marker protein is labeled, and to which the second labeled antibody does not bind, as a mesenchymal circulating tumor cell that comprises the defective APC protein.
[10] The detection method described in [9] above, wherein the mesenchymal marker protein is at least one selected from the group consisting of vimentin, N-cadherin, OB-cadherin, fibronectin, integrin A5b1, Snail, Slug, ETS1, α-SMA, Twist, FAP, FSP-1, SIP1, Goosecoid, LEF-1, and FOXC2.
[11] The detection method according to any one of [1] to [10] above, further comprising the steps of: labeling an epithelial marker protein of the circulating tumor cell; and detecting a cell to which the first labeled antibody and the second labeled antibody bind and in which the epithelial marker protein is labeled, as an epithelial cell comprising the wild-type APC protein.
[12] The detection method described in [11] above, wherein the epithelial marker protein is at least one selected from the group consisting of cytokeratin, EpCAM, E-cadherin, ZO-1, laminin-1, entactin, MUC1, desmoplakin, and α1 collagen.
[13] The detection method according to any one of the above-mentioned [1] to [12], wherein the first labeled antibody binds to a site present in both the wild-type APC protein and the defective APC protein, and the second labeled antibody binds to a site present in the wild-type APC protein but not in the defective APC protein.
[14] A method for detecting circulating tumor cell in a sample which comprises a defective APC protein to which a first labeled antibody is bound and a second labeled antibody is not bound, wherein the circulating tumor cell is prepared by contacting the circulating tumor cell in the sample with the first labeled antibody and the second labeled antibody, the first labeled antibody comprising a first labeling substance and an antibody capable of binding to a wild-type APC protein and a defective APC protein, and the second labeled antibody comprising a second labeling substance different from the first labeling substance and an antibody capable of binding to the wild-type APC protein and not to the defective APC protein.
[15] The detection method according to any one of [1] to [14] above, wherein the detection of the CTCs is carried out with a fluorescent microscope or a flow cytometer.

### [Effect of the invention]

According to the present invention, CTCs containing defective APC proteins can be detected.

### [Brief description of the drawings]

FIG. 1A is a schematic diagram of a wild-type APC protein bound to a first labeled antibody and a second labeled antibody.
FIG. 1B is a schematic diagram of a truncated APC protein bound to a first labeled antibody.
FIG. 1C is a schematic diagram of a truncated APC protein bound to a first labeled antibody.
FIG. 2A is a schematic diagram showing an example of a reagent kit according to the present embodiment.
FIG. 2B is a schematic diagram showing an example of a reagent kit according to the present embodiment.
FIG. 2C is a schematic diagram showing an example of a reagent kit according to the present embodiment.
FIG. 2D is a schematic diagram showing an example of a reagent kit according to the present embodiment.
FIG. 3 shows the results of detecting APC protein in A549 cells, HCT116 cells, and DLD1 cells by Western blotting.
Figure 4 shows an example of: a bright-field image obtained by immunostaining A549 cells, HCT116 cells, and DLD1 cells and measuring them with an imaging flow cytometer (IFCM); a fluorescent image (APC-N) derived from an Alexa 488-labeled anti-APC-N antibody; a fluorescent image (APC-C) derived from an unlabeled anti-APC-C antibody bound to an Alexa 647-labeled secondary antibody that recognizes the APC-C antibody (Alexa 647-labeled anti-APC-C antibody); and an image (Merge) where APC-N and APC-C are superimposed.
FIG. 5 shows the ratios of APC-C fluorescence intensity to APC-N fluorescence intensity (APC-C/APC-N ratios) obtained by measuring immunostained A549 cells, HCT116 cells, and DLD1 cells by IFCM.
FIG. 6A is a scattergram obtained by immunostaining DLD1 cells and measuring them with a flow cytometer (FCM). The vertical axis represents the fluorescence intensity (area) derived from APC-C, and the horizontal axis represents the fluorescence intensity (area) derived from APC-N.
FIG. 6B is a scattergram obtained by immunostaining HCT116 cells and measuring them by FCM. The vertical axis represents the fluorescence intensity (area) derived from APC-C, and the horizontal axis represents the fluorescence intensity (area) derived from APC-N.
FIG. 7 shows the APC-C/APC-N ratios obtained by immunostaining primary cultured cells (iCCs) derived from 13 colorectal cancer patients (Donors 1-4, 6-8, and 10-15) and measuring them by IFCM.
FIG. 8 shows examples of bright-field and fluorescent images obtained by IFCM measurement of iCCs from a colon cancer patient whose APC protein was determined to be defective and of iCCs from a colon cancer patient whose APC protein was determined to be wild-type.
FIG. 9 shows the results of detecting vimentin and GAPDH in HCC827 cells by Western blotting.
FIG. 10A is a scattergram obtained by immunostaining HCC827 cells that were not treated with TGF-β and measuring them by IFCM. The vertical axis represents the fluorescence intensity derived from the labeled anti-cytokeratin antibody, and the horizontal axis represents the fluorescence intensity derived from the labeled anti-vimentin antibody.
FIG. 10B is a scattergram obtained by immunostaining TGF-β-treated HCC827 cells and measuring them by IFCM. The vertical axis represents the fluorescence intensity derived from the labeled anti-cytokeratin antibody, and the horizontal axis represents the fluorescence intensity derived from the labeled anti-vimentin antibody.
FIG. 11A shows a scattergram obtained by immunostaining iCCs derived from Donor 1 and measuring them by IFCM. The vertical axis represents the fluorescence intensity derived from the labeled anti-cytokeratin antibody, and the horizontal axis represents the fluorescence intensity derived from the labeled anti-vimentin antibody.
FIG. 11B is a scattergram obtained by immunostaining iCCs derived from Donor 2 and measuring them by IFCM. The vertical axis represents the fluorescence intensity derived from the labeled anti-cytokeratin antibody, and the horizontal axis represents the fluorescence intensity derived from the labeled anti-vimentin antibody.
FIG. 11C is a scattergram obtained by immunostaining iCCs derived from Donor 12 and measuring them by IFCM. The vertical axis represents the fluorescence intensity derived from the labeled anti-cytokeratin antibody, and the horizontal axis represents the fluorescence intensity derived from the labeled anti-vimentin antibody.
FIG. 11D is a scattergram obtained by immunostaining iCCs derived from Donor 3 and measuring them by IFCM. The vertical axis represents the fluorescence intensity derived from the labeled anti-cytokeratin antibody, and the horizontal axis represents the fluorescence intensity derived from the labeled anti-vimentin antibody.
FIG. 12A is a scattergram obtained by immunostaining HCC827 cells that were not treated with TGF-β and measuring them by FCM. The vertical axis represents the fluorescence intensity (area) derived from the labeled anti-cytokeratin antibody, and the horizontal axis represents the fluorescence intensity (area) derived from the labeled anti-vimentin antibody.
FIG. 12B is a scattergram obtained by immunostaining TGF-β-treated HCC827 cells and measuring them by FCM. The vertical axis represents the fluorescence intensity (area) derived from the labeled anti-cytokeratin antibody, and the horizontal axis represents the fluorescence intensity (area) derived from the labeled anti-vimentin antibody.
FIG. 13 shows examples of bright-field and fluorescent images of epithelial CTCs, intermediate CTCs, and mesenchymal CTCs from a patient with colon cancer determined to be defective by IFCM measurement.
FIG. 14A shows the fluorescence intensity of APC-N measured by IFCM after immunostaining peripheral blood mononuclear cells (PBMCs) with Alexa 488-labeled anti-APC-N antibody and Alexa 647-labeled anti-APC-C antibody after storage at room temperature for 0, 24, or 48 hours.
FIG. 14B shows the fluorescence intensity of APC-C measured by IFCM after immunostaining PBMCs with Alexa 488-labeled anti-APC-N antibody and Alexa 647-labeled anti-APC-C antibody after storage at room temperature for 0, 24, or 48 hours.
FIG. 15A shows the fluorescence intensity measured by IFCM after storing HCC827 cells with and without epithelial-mesenchymal transition (EMT) induced at room temperature for 0, 24, or 48 hours and immunostaining with a labeled anti-vimentin antibody.
FIG. 15B shows the fluorescence intensity measured by IFCM after storing HCC827 cells with and without EMT induction at room temperature for 0, 24, or 48 hours and then immunostaining with a labeled anti-cytokeratin antibody.

### DETAILED DESCRIPTION OF THE PREFERRED EMBODIMENTS

In the CTC detection method of this embodiment (hereinafter also referred to as the "detection method of this embodiment"), first, CTCs in a sample are contacted with a first labeled antibody containing a first labeling substance, and a second labeled antibody containing a second labeling substance different from the first labeling substance. The detection method of this embodiment is carried out in vitro.

Since CTCs are generally present in blood, the sample is either blood drawn from a subject (whole blood) or a preparation from the blood. The whole blood is, for example, peripheral blood. If necessary, a known anticoagulant such as heparin, EDTA salt, or sodium citrate may be added to the whole blood. The whole blood may be diluted with a suitable aqueous medium if necessary. Such an aqueous medium is not particularly limited as long as it does not interfere with the antigen-antibody reaction described below, and examples thereof include water, physiological saline, and buffer solutions. The subject is not particularly limited, and examples include healthy subjects, cancer patients, and those suspected of having cancer. The type of cancer is not particularly limited. Since defective APC protein is a marker for colon cancer, samples derived from colon cancer patients are particularly suitable for the detection method of this embodiment.

When the sample is a preparation from whole blood, the preparation is preferably a fraction prepared from whole blood obtained from a subject and capable of containing CTCs. In this specification, "fractions that may contain CTCs" encompasses both fractions that contain CTCs and fractions that may contain CTCs. It is known in the art that blood collected from a cancer patient may not contain CTCs. Examples of fractions that may contain CTCs include fractions obtained by removing red blood cells from whole blood, fractions obtained by removing red blood cells and/or white blood cells from whole blood, and fractions obtained by selectively recovering CTCs from whole blood. Red blood cells can be removed, for example, by adding a hemolyzing agent to whole blood to cause hemolysis. Leukocytes can be removed, for example, by capturing the leukocytes using a solid phase (magnetic particles, microchannels, etc.) to which an anti-CD45 antibody has been immobilized. Since red blood cells and white blood cells account for the majority of cells in whole blood, a fraction that may contain CTCs can be obtained by removing red blood cells and white blood cells from whole blood. Selective recovery of CTCs from whole blood can be performed, for example, by using a commercially available CTC enrichment and recovery device such as the ClearCell (registered trademark) FX system or the On-Chip (registered trademark) Sort system, and a commercially available separation and enrichment chip such as the CTChip (registered trademark) FR1S. These methods allow the separation and recovery of fractions that may contain CTCs from whole blood. More preferably, the sample is obtained by separating whole blood into a fraction that may contain CTCs and a fraction that contains other cells, and recovering the fraction that may contain CTCs. The fraction that may contain CTCs and is thus separated and collected from whole blood is particularly suitable as a sample in the detection method of this embodiment. It is difficult to completely remove other cells, such as leukocytes, from a fraction that may contain CTCs. A fraction that may contain CTCs may contain small amounts of cells other than CTCs, such as white blood cells.

The sample may include fixed CTCs. Fixation treatment prevents protein degradation in CTCs. Fixation can be carried out by adding a fixative agent such as paraformaldehyde (PFA) or formaldehyde to a fraction that may contain CTCs. A commercially available cell fixative may also be used. The sample may include membrane-permeabilized CTCs. Membrane permeabilization allows the labeled antibody described below to pass through the cell membrane and enter the cells. Membrane permeabilization can be performed by adding a membrane permeabilization reagent, such as methanol, acetone, or a surfactant, to a fraction that may contain CTCs. Fixation and membrane permeabilization processes per se are known in the art. Preferably, the sample comprises fixed and permeabilized CTCs.

The APC protein is a protein comprising 2843 amino acid residues, encoded by the APC gene which was isolated as the gene responsible for familial adenomatous polyposis. The amino acid sequence of the APC protein itself is publicly known and is disclosed in public databases such as NCBI (National Center for Biotechnology Information). The APC protein is usually wild-type, but CTCs can contain defective APC protein. One of the functions of wild-type APC protein is known to be suppression of Wnt signaling by binding to β-catenin. As a result, excessive cell proliferation is prevented. Therefore, the normal APC gene is considered to be a tumor suppressor gene. Mutations in the APC gene can result in the expression of defective APC proteins. Mutations in the APC gene are particularly common in colorectal cancer. There are various defective APC proteins depending on the type and location of the gene mutation, but most of them are unable to bind to β-catenin. Thus, defective APC proteins are unable to regulate Wnt signaling, which can lead to uncontrolled cell proliferation that can lead to cancer development.

In this specification, a "defective APC protein" is one in which the amino acid sequence in the C-terminal region of the wild-type APC protein is absent, or which contains an amino acid sequence in the C-terminal region that differs from that of the wild-type APC protein. The C-terminal region can be a region that accounts for 30% to 60% of the entire amino acid sequence of the wild-type APC protein, including the C-terminal amino acid residues. For example, the C-terminal region can be the region from amino acid residue 1139 to the C-terminus (amino acid residue 2843) of the wild-type APC protein. In this case, the C-terminal region accounts for 59.9% of the entire amino acid sequence of the wild-type APC protein. Alternatively, the C-terminal region can be the region from amino acid residue 1991 to the C-terminus (amino acid residue 2843) of the wild-type APC protein. In this case, the C-terminal region corresponds to 30% of the entire amino acid sequence of the wild-type APC protein. Preferably, the C-terminal region is the region from amino acid residue 1200 to the C-terminus (amino acid residue 2843) of the wild-type APC protein. The location of gene mutations that lead to deletion of the C-terminal region of the APC protein is specifically described in Zhang Z. et al., ONCOLOGY LETTERS 19: 1781-1788, 2020, Zhang L. and Shay JW, JNCI J Natl Cancer Inst (2017) 109(8): djw332, etc.

When the amino acid sequence of the C-terminal region is absent, the defective APC protein has no C-terminal region. That is, the defective APC protein lacks the C-terminal region compared to the wild-type APC protein. When a defective APC protein contains an amino acid sequence in its C-terminal region that differs from that of a wild-type APC protein, the C-terminal region of the defective APC protein loses the function that is fulfilled by the C-terminal region of the wild-type APC protein. Such functions include, for example, the ability to bind to β-catenin, the ability to bind to microtubules, the ability to bind to EB1 protein, and the like. Naturally, defective APC proteins that do not have a C-terminal region also lose such functions. Mutations in the APC gene that can result in defective APC proteins include nonsense mutations resulting from base substitutions and frameshift mutations resulting from base insertions or deletions. Specifically, a mutation in the APC gene can result in the generation of a stop codon, which can result in only a portion of the transcript of the APC gene being translated. Alternatively, a portion of the APC gene may be deleted, leaving part of the gene itself absent. The defective APC proteins that arise in these cases lack the amino acid sequence of the C-terminal region. Furthermore, a frameshift mutation may occur in the APC gene, which may result in a change in the nucleotide sequence downstream of the mutation site. The C-terminal region of the defective APC protein generated in this case has an amino acid sequence different from that of the wild-type APC protein.

The CTCs in the sample may be at least one of i) CTCs that contain a defective APC protein and no wild-type APC protein, ii) CTCs that do not contain a defective APC protein and contain a wild-type APC protein, and iii) CTCs that contain both a defective APC protein and a wild-type APC protein. Preferably, the CTCs of i) and/or the CTCs of iii) are detected as "CTCs containing a defective APC protein". Furthermore, the CTCs in ii) above are detected as "CTCs containing a wild-type APC protein." When a sample contains at least one of the CTCs of i) and the CTCs of iii), CTCs containing a defective APC protein can be detected from the sample.

In the detection method of this embodiment, two types of labeled antibodies are used to detect CTCs containing a defective APC protein: a first labeled antibody containing a first labeling substance (hereinafter also simply referred to as the "first labeled antibody"), and a second labeled antibody containing a second labeling substance (hereinafter simply referred to as the "second labeled antibody"). In this specification, a "labeled antibody" refers to a complex containing a detection antibody and labeling substance. Specifically, the detection antibody that binds to the APC protein is an antibody that can bind to both the wild-type APC protein and the defective APC protein described below, and an antibody that can bind to the wild-type APC protein but not to the defective APC protein. The labeling substances for detecting the APC protein are the first labeling substance and the second labeling substance described below. The labeled antibody can be a detection antibody labeled with a labeling substance. Alternatively, the labeled antibody may be a complex comprising a detection antibody and a secondary antibody that specifically binds to the detection antibody and is labeled with a labeling substance.

Labeling of an antibody with a labeling substance is known in the art, and can be appropriately selected depending on the type of labeling substance. For example, a suitable cross-linking agent can be used to bind the detection antibody to the labeling substance. When the labeling substance is a protein, the first labeled antibody and/or the second labeled antibody may be a fusion protein of the labeling substance and a detection antibody. A detection antibody modified with biotin and a labeling substance modified with avidin may also be used. In this case, the labeling substance can indirectly bind to the detection antibody via the specific binding between biotins and avidins. Biotins include biotin and biotin analogues such as desthiobiotin and oxybiotin. The avidins include avidin and avidin analogues such as streptavidin and tamavidin (registered trademark).

In this specification, the term "antibody" encompasses full length antibodies and fragments thereof. The full-length antibody may be any of IgG, IgA, IgM, IgD and IgE, but is preferably IgG. Examples of antibody fragments include Fab, Fab', F(ab')2, Fd, Fd', Fv, light chains, heavy chain variable regions of heavy chain antibodies (VHH), reduced IgG (rIgG), and single chain antibodies (scFv). The antibody may be either a monoclonal antibody or a polyclonal antibody, with a monoclonal antibody being preferred. The antibody may be from any animal. Such animals are preferably mammals, and examples thereof include rabbits, mice, alpacas, camels, rats, pigs, sheep, goats, cows, horses, donkeys, and humans.

The first labeled antibody, as the detection antibody, includes an antibody capable of binding to both the wild-type APC protein and the defective APC protein. The detection antibody in the first labeled antibody can be an antibody that binds to a site present in both the wild-type and defective APC proteins. The second labeled antibody, as a detection antibody, comprises an antibody capable of binding to a wild-type APC protein and not to a defective APC protein. The detection antibody in the second labeled antibody can be an antibody that binds to a site that is present in the wild-type APC protein and not present in the defective APC protein. That is, the first labeled antibody and the second labeled antibody recognize different epitopes. Preferably, the epitope of the first labeled antibody is present at a site other than the C-terminal region of the wild-type APC protein, and the epitope of the second labeled antibody is present within the C-terminal region of the wild-type APC protein.

Contact between the CTCs in a sample, the first labeled antibody, and the second labeled antibody can be achieved, for example, by mixing the sample that may contain CTCs with a solution containing the first labeled antibody and a solution containing the second labeled antibody. Alternatively, CTCs in a sample may be immobilized on a solid phase capable of immobilizing CTCs, and then a solution containing the first labeled antibody and a solution containing the second labeled antibody may be added to the solid phase. In the contacting step, a solution containing both the first labeled antibody and the second labeled antibody may be used. The order in which the CTCs are contacted with the first labeled antibody and the second labeled antibody is not particularly limited, and they may be mixed simultaneously or sequentially.

When a CTC contains a defective APC protein and/or a wild-type APC protein, an antibody-antigen reaction occurs upon contact of the CTC with the first labeled antibody and the second labeled antibody. The reaction is usually carried out in an aqueous medium. The aqueous medium is not particularly limited, but examples thereof include water, saline, phosphate buffered saline (PBS), Tris buffered saline (TBS), Good's buffer solution, and the like. Examples of Good's buffer solutions include MES, Bis-Tris, ADA, PIPES, Bis-Tris-Propane, ACES, MOPS, MOPSO, BES, TES, HEPES, HEPPS, Tricine, Tris, Bicine, and TAPS.

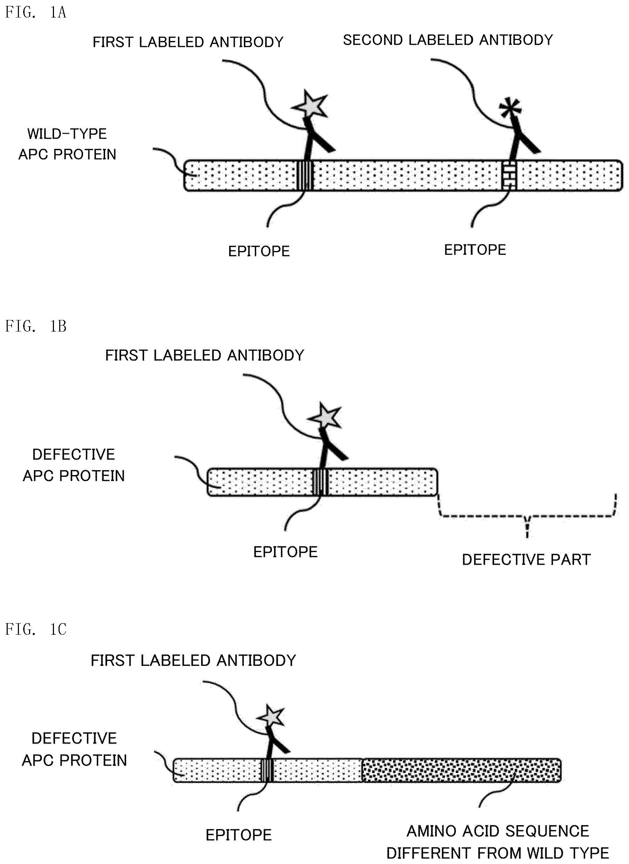

Through the above-mentioned antigen-antibody reaction, the APC protein contained in the CTCs is labeled with the labeled antibody. With reference to Figures 1A to 1C, labeling of APC proteins contained in CTCs will be described. FIG. 1A is a schematic diagram of a wild-type APC protein bound intracellularly to a first labeled antibody and a second labeled antibody. The wild-type APC protein has both the epitope of the first labeled antibody and the epitope of the second labeled antibody. Therefore, when a CTC containing a wild-type APC protein is contacted with the first labeled antibody and the second labeled antibody, both the first labeled antibody and the second labeled antibody bind to the wild-type APC protein. From cells containing a wild-type APC protein, it is possible to obtain both a signal derived from the first labeling substance carried by the first labeled antibody and a signal derived from the second labeling substance carried by the second labeled antibody.

FIG. 1B is a schematic diagram of a truncated APC protein with a first labeled antibody bound thereto within a cell.

In this example of the defective APC protein, the C-terminal region is deleted. As described above, this defective APC protein can arise due to a deletion of the APC gene, generation of a stop codon due to a mutation in the APC gene, or the like. In this truncated APC protein, the epitope for the second labeled antibody is lost. Therefore, the first labeled antibody binds to the defective APC protein, but the second labeled antibody does not bind to it. From cells containing the defective APC protein, a signal derived from the first labeling substance carried by the first labeled antibody is obtained, but a signal derived from the second labeling substance carried by the second labeled antibody is not obtained.

FIG. 1C is a schematic diagram of a truncated APC protein with a first labeled antibody bound thereto within a cell.

In this example, the defective APC protein contains an amino acid sequence in the C-terminal region that differs from that of the wild-type APC protein. As described above, this defective APC protein can occur due to a frameshift mutation in the APC gene. In this truncated APC protein, the epitope for the second labeled antibody is lost. Therefore, the first labeled antibody binds to the defective APC protein, but the second labeled antibody does not bind to it. From cells containing the defective APC protein, a signal derived from the first labeling substance carried by the first labeled antibody is obtained, but a signal derived from the second labeling substance carried by the second labeled antibody is not obtained.

Thus, there is a difference in the signals obtained between the defective APC protein and the wild-type APC protein. In the detection method of this embodiment, it is possible to identify whether the APC protein contained in each cell is a wild-type APC protein or a defective APC protein, based on this difference. That is, in the detection method of this embodiment, cells containing an APC protein to which the first labeled antibody binds but the second labeled antibody does not bind can be detected as cells containing a defective APC protein. Furthermore, in the detection method of this embodiment, cells containing an APC protein bound to the first labeled antibody and the second labeled antibody can be detected as cells containing a wild-type APC protein. Defective APC protein is known as a marker for colorectal cancer. Therefore, cells containing a defective APC protein in a sample that may contain CTCs can be determined to be CTCs.

The first labeled antibody comprises a first labeling substance and the second labeled antibody comprises a second labeling substance. The second labeling substance is different from the first labeling substance. Specifically, the first labeling substance and the second labeling substance are substances that generate signals different from each other. The signal derived from the first labeling substance is called the "first signal," and the signal derived from the second labeling substance is called the "second signal." The labeling substance is preferably a substance that generates a signal by itself (hereinafter also referred to as a "signal generating substance"). The first labeling substance and the second labeling substance may be the same type of signal generating substance, but it is preferable that the first signal and the second signal are distinguishably different. An example of a signal generating substance is a fluorescent substance.

The fluorescent substance is not particularly limited and can be appropriately selected from, for example, known fluorescent dyes and fluorescent proteins. Examples of fluorescent dyes include fluorescein isothiocyanate (FITC), rhodamine, coumarin, imidazole derivatives, indole derivatives, allophycocyanin, phycoerythrin (PE), PerCP/Cy5.5^{™}, Alexa Fluor^{®}, Cy3^{®}, Cy5^{®}, Cy5.5^{®}, Cy7^{®}, DyLight^{®} Fluor, and the like. Examples of fluorescent proteins include green fluorescent protein, yellow fluorescent protein, blue fluorescent protein, and red fluorescent protein. When both the first labeling substance and the second labeling substance are fluorescent substances, the first labeling substance and the second labeling substance are preferably fluorescent substances having fluorescence emission maxima in different wavelength ranges. That is, it is preferable that the wavelength of the maximum fluorescence emission of the first labeling substance is different from the wavelength of the maximum fluorescence emission of the second labeling substance. This allows each of the first signal and the second signal to be measured.

In a preferred embodiment, detection of CTCs containing defective APC proteins is performed by detecting a first signal and a second signal arising from individual cells. As shown in Figures 1B and 1C, the second signal is not detected from the defective APC protein. Therefore, cells whose second signal is equal to or lower than the threshold value can be determined to be CTCs containing a defective APC protein. Furthermore, cells in which the second signal is higher than a threshold value may be determined to be cells containing the wild-type APC protein. In a further embodiment, a ratio value of the first signal to the second signal is obtained. Based on the value of the ratio, CTCs containing defective APC proteins may be detected. For example, cells in which the ratio value of the second signal to the first signal (second signal/first signal) is less than a threshold value, or the ratio value of the first signal to the second signal (first signal/second signal) is equal to or greater than a threshold value, can be detected as CTCs containing a defective APC protein. Furthermore, a cell in which the ratio value of the second signal to the first signal (second signal/first signal) is equal to or greater than a threshold value, or the ratio value of the first signal to the second signal (first signal/second signal) is less than a threshold value, may be determined to be a cell containing a wild-type APC protein. The threshold value for each signal can be set independently by referring to Example 2 described below.

When the labeling substance is a fluorescent substance, the fluorescence emitted from the fluorescent substance can be measured using an apparatus such as a fluorescence microscope, FCM, or IFCM. In this specification, an FCM is a device that irradiates light onto individual particles (e.g., cells) in a liquid flowing through a flow cell and acquires optical information from each particle. An IFCM is an FCM equipped with an imaging unit such as a CCD camera. An IFCM can acquire images of individual cells in a liquid flowing through a flow cell, and can acquire and quantitatively measure optical information, fluorescent images, and bright-field images (also called transmitted light images) from several to several million cells in a short period of time, for example, from a few seconds to a few minutes. Furthermore, image processing can be used to extract information about individual cells.

The information obtained by measurement with FCM is the optical information of individual particles. An example of the optical information is fluorescent signal information. As the fluorescent signal information, for example, the fluorescent intensity based on the waveform of the fluorescent signal for each cell can be obtained. In this specification, the fluorescence intensity obtained by measurement with FCM may be the peak value, width, area, etc. of the waveform of the fluorescent signal. Here, the "area" of the fluorescent signal waveform is calculated by integrating the fluorescent signal waveform. In detecting CTCs containing a defective APC protein, the fluorescence intensities obtained by measurement with FCM can be used as the first signal and the second signal. The FCM is not particularly limited, and a commercially available device may be used. An example of a commercially available device is FACSVerse^{™} (Becton Dickinson Japan).

Detection of CTCs containing defective APC proteins may be performed by obtaining images of individual cells in a sample and based on the images of the cells. The image of the cell may be a fluorescent image of the cell. The means for acquiring a fluorescent image of a cell is not particularly limited. When the labeling substance is a fluorescent substance, a fluorescent image of the cells can be obtained using the above-mentioned fluorescence microscope, an IFCM capable of obtaining a fluorescent image, or other device.

The fluorescence microscope and the IFCM capable of acquiring fluorescent images are not particularly limited, and commercially available devices may be used. The light source is not particularly limited, and a light source having a wavelength suitable for exciting the fluorescent dye can be appropriately selected. As the light source, for example, a blue semiconductor laser, a red semiconductor laser, an argon laser, a He-Ne laser, a mercury arc lamp, or the like is used. Furthermore, the bright field light source for the IFCM or the like is not particularly limited, and for example, a white laser light source, an LED lamp, a mercury lamp, a xenon lamp, or the like may be used. Examples of commercially available systems include ImageStream MK II (Cytec Japan), DeNovo (bioview), Metafer (Metasystems), and MI-1000 (Sysmex Corporation).

When using a fluorescence microscope or an IFCM capable of acquiring fluorescence images, parameters including, for example, the fluorescence signal area value, the total fluorescence signal intensity (hereinafter, the total fluorescence signal intensity acquired by an IFCM or a fluorescence microscope is also simply referred to as "fluorescence intensity"), cell size, aspect ratio value, and the like can be acquired based on the acquired fluorescence image and transmitted light image. In detecting CTCs containing a defective APC protein, it is preferable to use at least the fluorescence intensity obtained by measurement with IFCM as the first signal and the second signal. The fluorescence intensity obtained by measurement with IFCM is the integral value of the pixel values of each pixel constituting an area showing a fluorescent signal in a fluorescent image of an individual cell. Particles with a total fluorescent signal intensity below a certain threshold can be determined to be cells to which the labeled antibody has not bound.

The first signal can be the fluorescence intensity derived from the first labeling substance, obtained by measurement with IFCM. The second signal can be the fluorescence intensity derived from the second labeling substance, obtained by measurement with IFCM. In this case, the ratio between the fluorescence intensities of the first signal and the second signal may be obtained as the ratio between the first signal and the second signal. As the ratio value between the first signal and the second signal, for example, first signal/second signal, second signal/first signal, etc. is obtained. Based on the value of this ratio, it may be possible to distinguish whether the APC protein is a wild-type APC protein or a defective APC protein. For example, if the second signal/first signal is below a threshold value, it can be determined that the cells in the sample (CTCs) contain a defective APC protein. Furthermore, if the second signal/first signal is equal to or greater than the threshold value, it can be determined that the cells in the sample contain the wild-type APC protein. The threshold may be, for example, 0.1, 0.2, 0.25, 0.3, 0.5, 0.75, 1.0, 1.5, 2.0 or 3.0. The ratio value between the first signal and the second signal may itself be used as an index. Alternatively, the ratio value between the first and second signals may be combined with another index or measurement.

The detection method of this embodiment may further include a step of labeling a mesenchymal marker protein and/or an epithelial marker protein, and a step of detecting CTCs labeled with the mesenchymal marker protein and/or the epithelial marker protein. CTCs are known to exist as epithelial CTCs, mesenchymal CTCs, and intermediate CTCs that are in the process of transformation. It has been suggested that cancer cells may undergo epithelial-mesenchymal transition (EMT), changing from epithelial properties to mesenchymal properties. It is known that cancer cells that have undergone EMT acquire migration and invasive properties and become circulating in the blood. For this reason, mesenchymal cancer cells are considered to be more prone to metastasis and to be more malignant than epithelial cancer cells. In the detection method of this embodiment including the above steps, by labeling a mesenchymal marker protein and/or an epithelial marker protein, it is possible to determine whether the cells in a sample are mesenchymal, epithelial, or intermediate. The cells to be determined are not limited to CTCs, and all cells contained in the sample can be determined.

Examples of mesenchymal marker proteins include vimentin, N-cadherin, OB-cadherin, fibronectin, integrin A5b1, Snail, Slug, ETS1, α-SMA, Twist, FAP, FSP-1, SIP1, Goosecoid, LEF-1, and FOXC2. Epithelial marker proteins include cytokeratin, EpCAM, E-cadherin, ZO-1, laminin-1, entactin, MUC1, desmoplakin, and α1 collagen.

The mesenchymal marker protein and the epithelial marker protein can be labeled by known labeling methods. Labeling of a mesenchymal marker protein can be carried out, for example, by contacting cells in a sample with a labeled antibody capable of binding to the mesenchymal marker protein. Similarly, labeling of epithelial marker proteins can be achieved by contacting cells in a sample with a labeled antibody capable of binding to the epithelial marker protein. Hereinafter, the labeled antibody capable of binding to a mesenchymal marker protein is also referred to as the "third labeled antibody," and the labeled antibody capable of binding to an epithelial marker protein is also referred to as the "fourth labeled antibody." The labeling substance contained in the third labeled antibody is also referred to as the "third labeling substance," and the labeling substance contained in the fourth labeled antibody is also referred to as the "fourth labeling substance." The third labeled antibody comprises, as a detection antibody, an antibody capable of binding to a mesenchymal marker protein. The fourth labeled antibody comprises an antibody capable of binding to an epithelial marker protein as a detection antibody.

The third labeling substance and the fourth labeling substance may be any signal generating substance, for example, a fluorescent substance. Hereinafter, the signal derived from the third labeling substance is referred to as the "third signal," and the signal derived from the fourth labeling substance is referred to as the "fourth signal." When labeling a mesenchymal marker protein, the first labeling substance, the second labeling substance, and the third labeling substance may be the same type of signal generating substance, but it is preferable that the first signal, the second signal, and the third signal are each distinguishably different. When labeling an epithelial marker protein, the first labeling substance, the second labeling substance, and the fourth labeling substance may be the same type of signal generating substance, but it is preferable that the first signal, the second signal, and the fourth signal are each distinguishably different. When labeling both a mesenchymal marker protein and an epithelial marker protein, the first labeling substance, the second labeling substance, the third labeling substance and the fourth labeling substance may be the same type of signal generating substance, but it is preferable that the first signal, the second signal, the third signal and the fourth signal are each distinguishably different.

When a mesenchymal marker protein is labeled and the first labeling substance, the second labeling substance, and the third labeling substance are fluorescent substances, it is preferable that the wavelength of the fluorescence emission maximum of the first labeling substance, the wavelength of the fluorescence emission maximum of the second labeling substance, and the wavelength of the fluorescence emission maximum of the third labeling substance are different from each other. When an epithelial marker protein is labeled and the first labeling substance, the second labeling substance, and the fourth labeling substance are fluorescent substances, it is preferable that the wavelength of the fluorescence emission maximum of the first labeling substance, the wavelength of the fluorescence emission maximum of the second labeling substance, and the wavelength of the fluorescence emission maximum of the fourth labeling substance are different from each other. When both a mesenchymal marker protein and an epithelial marker protein are labeled and the first labeling substance, the second labeling substance, the third labeling substance, and the fourth labeling substance are fluorescent substances, it is preferable that the wavelength of the fluorescence emission maximum of the first labeling substance, the wavelength of the fluorescence emission maximum of the second labeling substance, the wavelength of the fluorescence emission maximum of the third labeling substance, and the wavelength of the fluorescence emission maximum of the fourth labeling substance are different from each other.

The third signal and/or the fourth signal can be detected as such in the same manner as the first signal and the second signal. When the third labeling substance and/or the fourth labeling substance is a fluorescent substance, the third signal and/or the fourth signal can be detected by FCM, IFCM or a fluorescence microscope. In a preferred embodiment, determining whether a cell in a sample is mesenchymal, epithelial or intermediate is made by detecting a third signal and/or a fourth signal arising from an individual cell. For example, when a mesenchymal marker protein is labeled, cells in which the third signal is equal to or greater than a threshold value can be determined to be mesenchymal cells. When an epithelial marker protein is labeled, cells in which the fourth signal is equal to or greater than a threshold value can be determined to be epithelial cells. When both a mesenchymal marker protein and an epithelial marker protein are labeled, a cell in which the third signal is equal to or greater than the threshold and the fourth signal is less than the threshold can be determined to be a mesenchymal cell. Furthermore, cells in which the third signal is below the threshold and the fourth signal is equal to or greater than the threshold can be determined to be epithelial cells. Furthermore, a cell in which the third signal is equal to or higher than a threshold and the fourth signal is equal to or higher than a threshold can be determined to be an intermediate cell. The threshold value for each signal can be set independently by referring to Example 3 described below.

Mesenchymal and epithelial marker proteins can also be expressed in CTCs. Therefore, by combining the results of detection of the APC protein using the first and second labeled antibody with the results of detection of the marker protein using the third and/or fourth labeled antibody for a single cell, it is possible to determine whether the single cell is a mesenchymal, epithelial, or intermediate CTC. For example, a cell in which the second signal is equal to or lower than a threshold value and the third signal is equal to or higher than a threshold value can be determined to be a mesenchymal CTC containing a defective APC protein. Alternatively, cells in which the second signal is higher than a threshold value and the third signal is equal to or greater than a threshold value may be determined to be mesenchymal cells (or CTCs) containing a wild-type APC protein. Furthermore, cells in which the second signal is equal to or lower than the threshold value and the fourth signal is equal to or higher than the threshold value can be determined to be epithelial CTCs containing a defective APC protein. Alternatively, cells in which the second signal is higher than a threshold value and the fourth signal is equal to or greater than a threshold value may be determined to be epithelial cells (or CTCs) containing a wild-type APC protein. Furthermore, a cell in which the second signal is equal to or lower than the threshold, the third signal is equal to or higher than the threshold, and the fourth signal is equal to or higher than the threshold can be determined to be an intermediate CTC containing a defective APC protein. Alternatively, cells in which the second signal is higher than a threshold, the third signal is equal to or higher than a threshold, and the fourth signal is equal to or higher than a threshold may be determined to be intermediate lineage cells (or CTCs) containing a wild-type APC protein.

In a further embodiment, cells in which the ratio value of the second signal to the first signal (second signal/first signal) is less than a threshold value or the ratio value of the first signal to the second signal (first signal/second signal) is equal to or greater than a threshold value and the third signal is equal to or greater than a threshold value can be determined to be mesenchymal CTCs containing a defective APC protein. Alternatively, cells in which the second signal/first signal is equal to or greater than a threshold value, or the first signal/second signal is less than a threshold value and the third signal is equal to or greater than a threshold value, may be determined to be mesenchymal cells (or CTCs) containing wild-type APC protein.

In a further embodiment, a cell in which the second signal/first signal is below a threshold value or the first signal/second signal is equal to or above a threshold value and the fourth signal is equal to or above a threshold value can be determined to be an epithelial CTC containing a defective APC protein. Alternatively, a cell in which the second signal/first signal is equal to or greater than a threshold value, or the first signal/second signal is less than a threshold value and the fourth signal is equal to or greater than a threshold value, may be determined to be an epithelial cell (or CTC) containing a wild-type APC protein.

In a further embodiment, a cell in which the second signal/first signal is below a threshold value or the first signal/second signal is above a threshold value, the third signal is above a threshold value, and the fourth signal is above a threshold value can be determined to be an intermediate CTC containing a defective APC protein. Alternatively, a cell in which the second signal/first signal is above a threshold or the first signal/second signal is below a threshold, the third signal is above a threshold, and the fourth signal is above a threshold may be determined to be an intermediate cell (or CTC) containing a wild-type APC protein.

A further embodiment of the invention is a method of detecting CTCs that contain a defective APC protein to which the first labeled antibody is bound and the second labeled antibody is not bound. CTCs containing a defective APC protein to which the first labeled antibody is bound and the second labeled antibody is not bound are prepared by contacting CTCs in a sample with the first labeled antibody and the second labeled antibody. The first labeled antibody comprises a first labeling substance and an antibody capable of binding to the wild-type APC protein and the defective APC protein. The second labeled antibody comprises a second labeling substance different from the first labeling substance, and an antibody capable of binding to the wild-type APC protein but not to the defective APC protein. The labeling of CTCs prepared by contacting a first labeled antibody with a second labeled antibody is measured, and defective APC protein to which the first labeled antibody binds but not the second labeled antibody is detected, thereby indicating that the sample contains CTCs containing defective APC protein. The method for detecting the APC protein of a CTC, the first labeled antibody, the labeling substance, the CTC, and the APC protein are as described above.

The above detection method may involve contacting the CTCs in the sample with a third labeled antibody and/or a fourth labeled antibody. The mesenchymal marker protein, epithelial marker protein, and respective labeled antibodies are as described above. Each of these antibodies is preferably labeled with a labeling substance. The labeling substance is as described above. By confirming whether an antibody that labels a mesenchymal marker protein or an epithelial marker protein is bound, it is possible to determine whether a CTC is mesenchymal or epithelial.

When a sample collected from a subject contains a predetermined amount or more of CTCs containing a defective APC protein, the subject or the sample can be determined to be "defective APC protein positive." If a sample collected from a subject contains less than a predetermined amount of CTCs containing defective APC protein, or if no CTCs containing defective APC protein are detected, the subject or sample can be determined to be "negative for defective APC protein." In a preferred embodiment, if a sample collected from a subject contains less than a predetermined amount of CTCs containing defective APC protein, or no CTCs containing defective APC protein are detected, but the sample contains a predetermined amount or more of CTCs containing wild-type APC protein, the subject or sample can be determined to be "negative for defective APC protein."

The "predetermined amount" which is the threshold for determining that a defective APC protein is positive can be set appropriately. For example, detection can be performed in advance using multiple samples that have been found to be positive for defective APC protein by base sequence analysis or the like, and multiple samples that have been found to be negative for defective APC protein, and the above-mentioned "predetermined amount" can be a value that can appropriately classify defective APC protein positivity and negativity using indicators such as positive predictive value, negative predictive value, sensitivity, and specificity. Detection is performed on a specimen for which it is unknown whether it is negative or positive for the defective APC protein, and the predetermined amount is applied, thereby making it possible to determine whether the specimen is negative or positive for the defective APC protein.

It is also conceivable that a sample collected from a subject contains a predetermined amount or more of CTCs containing defective APC protein and a predetermined amount or more of CTCs containing wild-type APC protein. In this case, the genotype of the APC gene of the subject may be determined to be a heterozygote between the defective type and the wild type.

The reagents used in the above-mentioned detection methods can be provided to the user in the form of a reagent kit. The reagent kit includes a first labeled antibody and a second labeled antibody. The first labeled antibody and the second labeled antibody are as described above.

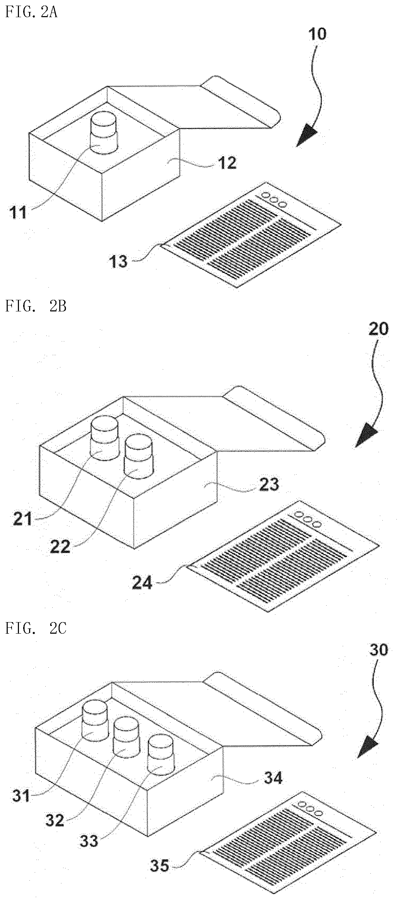

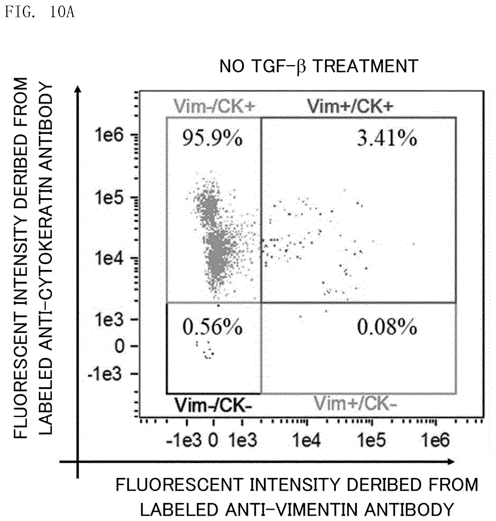

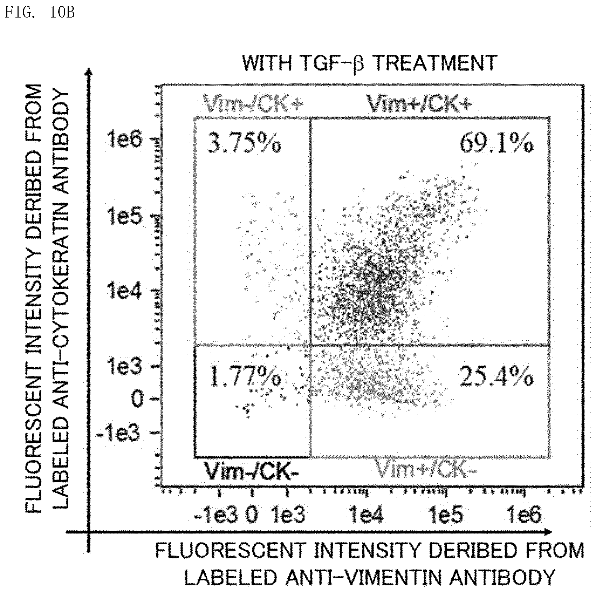

The reagent kit is composed of containers containing the respective reagents and a box containing the containers. The box may also contain a package insert. The package insert may include information about the configuration of the reagent kit, the composition of each reagent, and instructions for use. An example of the reagent kit of this embodiment is shown in FIG. 2A. In FIG. 2A, 10 indicates a reagent kit, 11 indicates a container containing a reagent including a first labeled antibody and a second labeled antibody, 12 indicates a packaging box, and 13 indicates an attached document. In this example, the first labeled antibody and the second labeled antibody are contained in the same container as one reagent.

Another example of the reagent kit is shown in FIG. 2B. In Figure 2B, 20 indicates a reagent kit, 21 indicates a first container containing a reagent including a first labeled antibody, 22 indicates a second container containing a reagent including a second labeled antibody, 23 indicates a packaging box, and 24 indicates an attached document. In this example, the first labeled antibody and the second labeled antibody are contained in separate containers.

The reagent kit may further include a reagent comprising a third labeled antibody that labels a mesenchymal marker protein or a fourth labeled antibody that labels an epithelial marker protein. An example of the reagent kit is shown in FIG. 2C. In Figure 2C, 30 indicates a reagent kit, 31 indicates a first container containing a reagent including a first labeled antibody, 32 indicates a second container containing a reagent including a second labeled antibody, 33 indicates a third container containing a reagent including a third labeled antibody or a fourth labeled antibody, 34 indicates a packaging box, and 35 indicates an attached document.

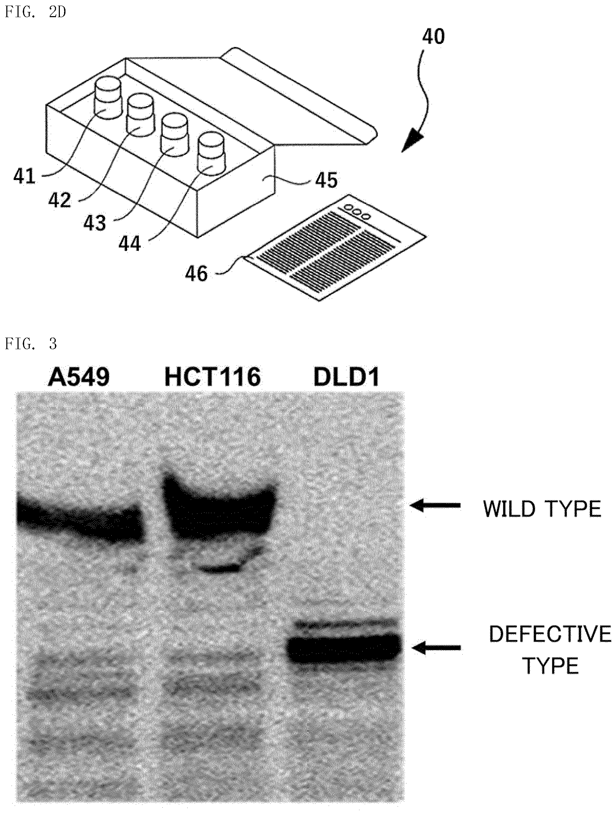

The reagent kit may further include a reagent containing a third labeled antibody, and a reagent containing a fourth labeled antibody. An example of the reagent kit is shown in FIG. 2D. In Figure 2D, 40 indicates a reagent kit, 41 indicates a first container containing a reagent including a first labeled antibody, 42 indicates a second container containing a reagent including a second labeled antibody, 43 indicates a third container containing a reagent including a third labeled antibody, 44 indicates a fourth container containing a reagent including a fourth labeled antibody, 45 indicates a packaging box, and 46 indicates an attached document.

The present invention will be described in detail below with reference to examples, but the present invention is not limited to these examples.

### EXAMPLES

### Example 1: Detection of cultured cells expressing defective APC protein

In order to establish an assay system for detecting CTCs expressing defective APC proteins, we first investigated an assay system capable of detecting cultured cells expressing defective APC proteins.

### (1) Cells and antibodies

As cell lines expressing the APC protein, the lung cancer-derived cell line A549, and the colon cancer-derived cell lines HCT116 and DLD1 were purchased from ATCC. A549 and HCT116 cells were known to express wild-type (ie, full-length) APC protein. DLD1 cells were known to express a defective APC protein in which the amino acid residues from amino acid 1427 to the C-terminus of wild-type APC were deleted. In the Western blot described below, anti-APC-N antibody (Santacruz: sc-53165 AF488) was used as an antibody that recognizes the N-terminus of the wild-type APC protein. In the immunostaining described below, Alexa 488-labeled anti-APC-N antibody (Santacruz: sc-53165 AF488) was used as a labeled antibody that recognizes the N-terminus of the wild-type APC protein. In addition, a combination of an unlabeled anti-APC-C antibody (Millipore: MAB3786) and an Alexa 647-labeled secondary antibody (Abeam: ab150115) that recognizes the unlabeled anti-APC-C antibody was used as a labeled antibody that recognizes the C-terminus of the wild-type APC protein. The site recognized by the anti-APC-C antibody was not present in the defective APC protein expressed by DLD1 cells. That is, it was predicted that the anti-APC-C antibody would not bind to the defective APC protein expressed by DLD1 cells.

### (2) Confirmation of APC protein expression in each cell line

Each of the A549 cells, HCT116 cells and DLD1 cells was lysed with a solubilizing agent containing a surfactant. The lysed cells were centrifuged to obtain the supernatant. A commercially available sample buffer was added to the supernatant, and the resulting mixture was boiled for 5 min. The heat-denatured mixture was separated by SDS-PAGE in a standard manner, and the proteins in the gel were transferred to a nitrocellulose membrane. This membrane was blocked with a blocking buffer (Nacalai Tesque: 03953-95), and Western blotting was performed using an anti-APC-N antibody and an HRP-labeled anti-mouse IgG antibody (MLB Life Sciences: 330). The results are shown in Figure 3. As can be seen from FIG. 3, bands derived from the anti-APC-N antibody were confirmed in all lanes. Compared with the APC proteins detected in A549 and HCT116 cells, the APC proteins detected in DLD1 cells had a smaller molecular weight. This result was consistent with reports that A549 and HCT116 cells express wild-type APC protein, whereas DLD1 cells express defective APC protein.

### (3) Cell culture and immunostaining

The above cells were cultured by conventional methods. The cells were harvested, washed with PBS, and then resuspended in 4% paraformaldehyde.

The cells were fixed by adding perfluoroalkane (PFA) solution. The fixed cells were washed with PBS containing 0.2% Pluronic F-127. The fixed cells were permeabilized with cold methanol at a final concentration of 90%. The permeabilized cells were washed with PBS containing 0.2% Pluronic F-127. Fixed and permeabilized cells were subjected to immunostaining using three types of antibodies. The immunostaining procedure was as follows.

First, PBS containing 5% BSA containing unlabeled anti-APC-C antibody was added, and the mixture was incubated at room temperature for 1 hour. After incubation, the cells were washed twice with PBS containing 0.2% Pluronic F-127. Next, PBS containing 5% BSA containing Alexa 647-labeled secondary antibody (Abeam: ab150115) that recognizes unlabeled anti-APC-C antibody was added, and the mixture was further incubated at room temperature for 30 minutes. After incubation, the cells were washed twice with PBS containing 0.2% Pluronic F-127. Then, PBS containing 5% BSA containing Alexa 488-labeled anti-APC-N antibody was added to the washed cells, and the cells were incubated at room temperature for 1 hour. After incubation, the cells were washed twice with PBS containing 0.2% Pluronic F-127. This resulted in a sample containing immunostained cells. In the following experiments, the unlabeled anti-APC-C antibody bound to an Alexa 647-labeled secondary antibody that recognizes the unlabeled anti-APC-C antibody is referred to as "Alexa 647-labeled anti-APC-C antibody."

### (4) Measurement by IFCM

The samples containing the immunostained cells were measured using an IFCM, ImageStream MK II (Cytec Japan). The measurements involved acquiring bright field and fluorescent images of individual particles in the sample. The acquired image data was analyzed using image analysis software IDEAS Application v6.2 (MERCK MILLIPORE). Images of 5,000 to 10,000 particles were obtained from the images captured with the ImageStream MK II. The particles in the acquired images were the subject of analysis. The fluorescence images of the particles to be analyzed were analyzed using the Intensity feature of IDEAS, and the total fluorescence signal intensity (fluorescence intensity) of Alexa 488 and Alexa 647 was obtained.

### (5) Detection of APC protein

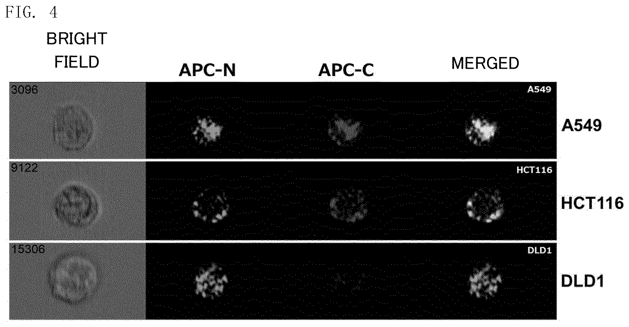

Examples of images obtained for A549 cells, HCT116 cells, and DLD1 cells are shown in FIG. In FIG. 4, "APC-N" indicates a fluorescent image derived from an Alexa 488-labeled anti-APC-N antibody, and "APC-C" indicates a fluorescent image derived from an Alexa 647-labeled anti-APC-C antibody. As can be seen from FIG. 4, in the fluorescent images of A549 cells and HCT116 cells, fluorescence derived from each labeled antibody was detected within the outlines of the cells confirmed in the bright field images. On the other hand, in the fluorescence images of DLD1 cells, fluorescence from the Alexa 488-labeled anti-APC-N antibody was detected within the cell outline confirmed in the bright-field images, but fluorescence from the Alexa 647-labeled anti-APC-C antibody was not detected. This suggested that the anti-APC-C antibody was unable to bind to the APC protein expressed in DLD1 cells. This result, together with the Western blot results in (2) above, demonstrated that cells expressing wild-type APC protein and cells expressing defective APC protein can be detected by measuring cells immunostained with labeled anti-APC-N antibody and anti-APC-C antibody by IFCM.

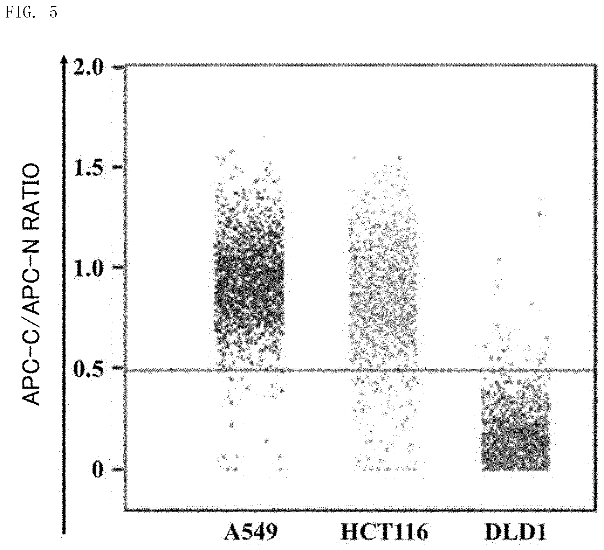

To analyze the trend of APC protein expression in all cells contained in a sample, the ratio of the fluorescence intensity derived from Alexa 647-labeled anti-APC-C antibody to the fluorescence intensity derived from Alexa 488-labeled anti-APC-N antibody (also referred to as the "APC-C/APC-N ratio value") was calculated for each cell in the sample. The APC-C/APC-N ratios were compared among samples containing A549 cells, HCT116 cells, and DLD1 cells. The results are shown in Figure 5. The dots in the figure indicate the APC-C/APC-N ratio values of individual cells. As can be seen from FIG. 5, a large difference was observed in the APC-C/APC-N ratio between the samples containing A549 cells and HCT116 cells and the samples containing DLD1 cells. These results demonstrated that cells expressing wild-type APC protein could be distinguished from cells expressing defective APC protein based on the fluorescence intensity from each of the fluorescently labeled anti-APC-N and anti-APC-C antibodies.

### (6) Measurement by FCM

HCT116 cells and DLD1 cells were immunostained in the same manner as described above (3). The samples containing the immunostained cells were measured using FACS Verse (Becton Dickinson Japan), an FCM. The measurement data was analyzed by a conventional method to obtain the intensities (fluorescence intensities) of the fluorescent signals generated by each of Alexa 488 and Alexa 647. A scattergram was prepared in which the vertical axis represents the fluorescence intensity derived from the Alexa 647-labeled anti-APC-C antibody and the horizontal axis represents the fluorescence intensity derived from the Alexa 488-labeled anti-APC-N antibody.

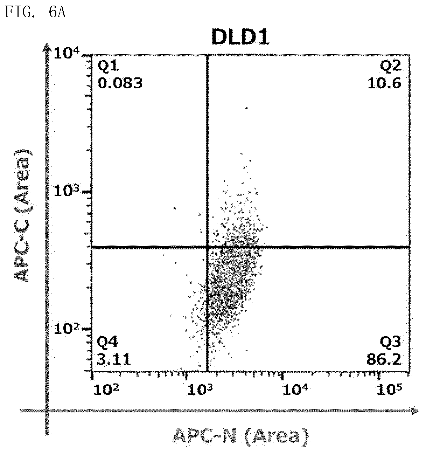

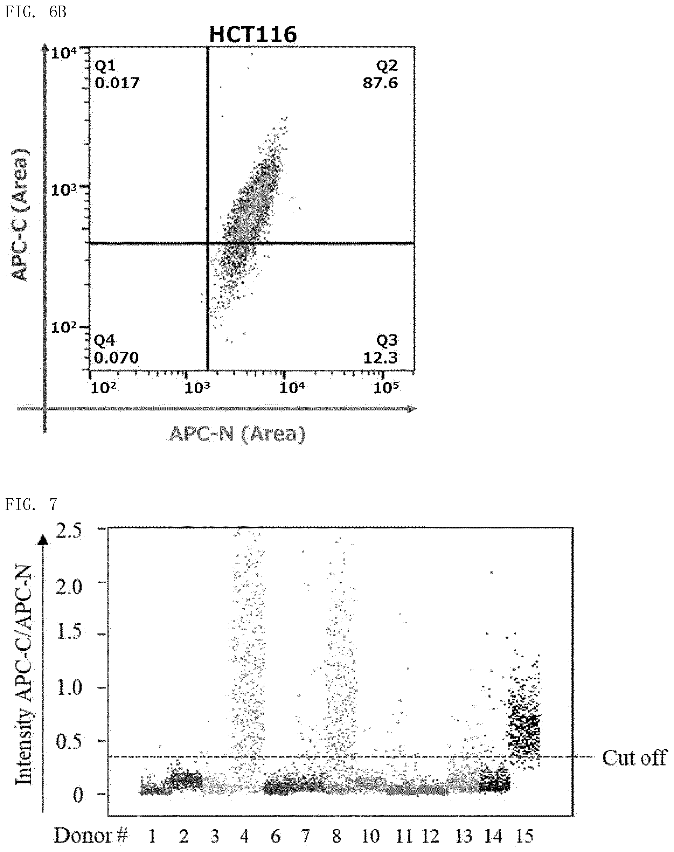

A scattergram of immunostained DLD1 cells is shown in FIG. 6A, and a scattergram of immunostained HCT116 cells is shown in FIG. 6B. In FIG. 6, "APC-N (Area)" indicates the fluorescence intensity (area) derived from the Alexa 488-labeled anti-APC-N antibody, and "APC-C (Area)" indicates the fluorescence intensity (area) derived from the Alexa 647-labeled anti-APC-C antibody. By comparing the scattergrams in Figures 6A and 6B, thresholds were set for the fluorescence intensities derived from the Alexa 647-labeled anti-APC-C antibody and the Alexa 488-labeled anti-APC-N antibody, respectively. In each scattergram, the vertical line represents the threshold value for Alexa 488-labeled anti-APC-N antibody, and the horizontal line represents the threshold value for Alexa 647-labeled anti-APC-C antibody. As can be seen from Figure 6A, in the scattergram of DLD1 cells, strong fluorescence intensity derived from the Alexa 488-labeled anti-APC-N antibody was detected, while cells with weak fluorescence intensity derived from the Alexa 647-labeled anti-APC-C antibody were mainly detected (bottom right of Figure 6A; 86.2% of the total). On the other hand, referring to Figure 6B, in the scattergram of HCT116 cells, cells in which the fluorescence intensity derived from both the Alexa 647-labeled anti-APC-C antibody and the Alexa 488-labeled anti-APC-N antibody exceeded the threshold were mainly detected (upper right of Figure 6B; 87.6% of the total). Thus, the distribution of cells in the scattergrams of immunostained DLD1 cells and those in the scattergrams of immunostained HCT116 cells was significantly different. These results demonstrated that FCM can also be used to distinguish between cells expressing wild-type APC protein and cells expressing defective APC protein based on the fluorescence intensity from Alexa 647-labeled anti-APC-C antibody and Alexa 488-labeled anti-APC-N antibody. In addition, the sensitivity and specificity of the FCM measurement results were 89% and 88%, respectively.

### Example 2: Detection of primary cultured cells expressing defective proteins

Primary culture cells prepared from tumor tissue were subjected to immunostaining and IFCM measurement in the same manner as in Example 1 to examine whether cells containing the APC protein could be detected.

### (1) Cells and antibodies

Tumor tissue was collected from each of 13 colon cancer patients (see FIG. 7). Primary cultured cells (isolated tumor-derived cancer cells, also called iCCs) were prepared from each patient's tumor tissue using standard methods. Genomic DNA was extracted from each iCC and sequenced to determine whether the APC gene contained a mutation that would result in a C-terminal deletion of the APC protein. Sequence analysis revealed that in Donors 1, 2, 6, 7, 11, 12, and 14, stop codons were detected in the APC gene due to mutations. In Donor 3, a frameshift was detected due to a mutation in the APC gene. In Donor 4, both the wild-type nucleotide sequence and a stop codon were detected. In Donor 8, both the wild-type sequence and a frameshift were detected. The genotype of the APC gene in Donors 4 and 8 was considered to be heterozygous between the defective type and the wild type. In Donor 10, both a stop codon and a frameshift were detected. In donors 13 and 15, wild-type nucleotide sequences were detected. In the immunostaining described below, the same labeled antibody as in Example 1 was used.

### (2) Immunostaining, IFCM measurement, and detection of APC protein

In the same manner as in Example 1, iCCs from each patient were immunostained to prepare samples. For immunostaining, in addition to the above antibodies, Hoechst (registered trademark) 33342 was used to stain nuclei. In the same manner as in Example 1, a sample containing immunostained iCCs was measured by IFCM. The APC-C/APC-N ratio was calculated for each patient's sample containing iCCs. The results are shown in Figure 7. The cutoff value for the APC-C/APC-N ratio was set at 0.25, and iCCs below this cutoff were determined to be CTCs containing defective APC protein, while iCCs above this cutoff value were determined to be cells containing wild-type APC protein. As can be seen from FIG. 7, in the sample containing iCCs from Donor 15, most of the iCCs had APC-C/APC-N ratio values higher than the cutoff and were determined to be cells containing the wild-type APC protein. This was consistent with the results of base sequence analysis. In the sample containing iCCS from Donor 4 and the sample containing iCCS from Donor 8, both cells containing wild-type APC protein and CTCs containing defective APC protein were detected. This was consistent with the results of base sequence analysis. With respect to other samples containing Donor's iCCs, most iCCs had an APC-C / APC-N ratio value lower than the cutoff, and were determined to be CTCs containing defective APC proteins. With the exception of Donor 13, the results were consistent with the results of nucleotide sequence analysis.

Furthermore, except for Donors 4 and 8, which were determined to be heterozygotes, the cutoff for the average APC-C/APC-N ratio value of particles in the samples was set to 0.25, and samples below this cutoff were determined to be "deficient type" and samples above this cutoff were determined to be "wild type." In this case, the samples from Donors 1, 2, 3, 6, 7, 10, 11, 12, 13, and 14 were determined to be of the defective type, and the sample from Donor 15 was determined to be of the wild type. Except for Donor 13, the results were consistent with the base sequence analysis. The above results demonstrate that patient samples expressing wild-type APC protein can be distinguished from patient samples expressing defective APC protein based on the fluorescence intensity derived from each of the fluorescently labeled anti-APC-N and anti-APC-C antibodies.

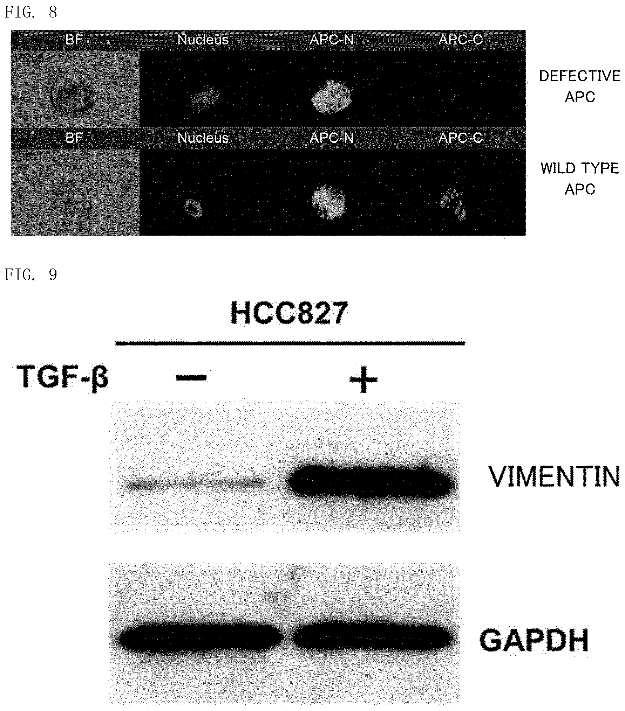

FIG. 8 shows examples of images obtained for iCCs from a patient determined to be defective by IFCM measurement and for iCCs from a patient determined to be wild-type. As can be seen from FIG. 8, in the fluorescence image of the wild type, fluorescence originating from each labeled antibody was detected. On the other hand, in the defective type fluorescence image, the fluorescence derived from the Alexa 488-labeled anti-APC-N antibody was detected, but the fluorescence derived from the Alexa 647-labeled anti-APC-C antibody was not detected. Therefore, it was demonstrated that iCCs expressing wild-type APC protein and iCCs expressing defective APC protein can be detected by measuring iCCs immunostained with labeled anti-APC-N and anti-APC-C antibodies by IFCM.

### Example 3: Discrimination of epithelial, mesenchymal or intermediate cells

We established a method for determining whether a cell is epithelial, mesenchymal, or intermediate, based on the signal values of an epithelial cell marker and a mesenchymal cell marker.

### (1) Cells and antibodies

The lung adenocarcinoma-derived cell line HCC827 was used in a preliminary experiment to determine the threshold value. HCC827 cells are normally epithelial cells, but it was known that stimulation with TGF-β induces EMT, causing them to become intermediate or mesenchymal cells. In addition, iCCs derived from 15 colon cancer patients prepared in Example 2 were also used. In the Western blot described below, an anti-vimentin antibody (BD Pharmingen: 550513) and an anti-GAPDH antibody (TREVIGEN: 2275-PC-100) were used. In the immunostaining described below, eFluor 615-labeled anti-cytokeratin antibody (Thermo Fisher: 42-9003-82) and PE-labeled anti-vimentin antibody (Santacruz: sc-6260 PE) were used.

### (2) Confirmation of epithelial-mesenchymal transition in HCC827 cells

HCC827 cells were cultured by conventional methods, and TGF-β was added to the culture medium to induce EMT. We also prepared HCC827 cells cultured without the addition of TGF-β. Each type of cell was lysed with a solubilizing agent containing a detergent. As in Example 1, samples were prepared from the lysed cells, separated by SDS-PAGE, and proteins were transferred to a nitrocellulose membrane. Western blotting was performed using anti-vimentin antibody and HRP-labeled anti-mouse IgG antibody (MLB Life Sciences, 330). The results are shown in Figure 9. As can be seen from FIG. 9, it was confirmed that the band derived from vimentin was increased by TGF-β treatment. These results demonstrated that TGF-β treatment induced EMT in HCC827 cells.

### (3) Immunostaining of HCC827 cells, IFCM measurement, and threshold determination

HCC827 cells were cultured by conventional methods, and TGF-β was added to the culture medium to induce EMT. We also prepared HCC827 cells cultured without the addition of TGF-β. The cells were harvested and washed with PBS, and then 4% PFA solution was added to fix the cells. The fixed cells were washed with PBS containing 0.2% Pluronic F-127. The fixed cells were permeabilized with cold methanol at a final concentration of 90%. The permeabilized cells were washed with PBS containing 0.2% Pluronic F-127. To the fixed and permeabilized cells, eFluor 615-labeled anti-cytokeratin antibody and PE-labeled anti-vimentin antibody in PBS containing 5% BSA were added, and the cells were incubated at room temperature for 1 hour. The cells were then washed twice with PBS. This resulted in a sample containing immunostained cells. The sample was measured using an ImageStream MK II (Cytec Japan). The measurements involved acquiring bright field and fluorescent images of individual particles in the sample. The acquired image data was analyzed using IDEAS Application v6.2 (MERCK MILLIPORE). Images of 5,000 to 10,000 particles were obtained from the images captured with the ImageStreem Mk II. The particles in the acquired images were analyzed. The fluorescent images of the particles to be analyzed were analyzed using the Intensity feature of IDEAS, and the intensities (fluorescence intensities) of the fluorescent signals generated by eFluor 615 and PE were obtained. A scattergram was prepared in which the vertical axis represents the fluorescence intensity derived from the eFluor 615-labeled anti-cytokeratin antibody and the horizontal axis represents the fluorescence intensity derived from the PE-labeled anti-vimentin antibody.

A scattergram of cells not treated with TGF-β is shown in FIG. 10A, and a scattergram of cells treated with TGF-β is shown in FIG. 10B. The scattergrams in FIGS. 10A and 10B were compared, and threshold values were set for the fluorescence intensities derived from the labeled anti-vimentin antibody and the labeled anti-vimentin antibody, respectively. In each scattergram, the vertical line represents the vimentin threshold and the horizontal line represents the cytokeratin threshold. This showed that the particles on the scattergram could be classified into three groups: epithelial cells (vimentin negative and cytokeratin positive: Vim-/CK+), mesenchymal cells (vimentin positive and cytokeratin negative: Vim+/CK-), and intermediate cells (vimentin positive and cytokeratin positive: Vim+/CK+). Specifically, referring to FIG. 10A, 95.9% of the cells not treated with TGF-β were classified as epithelial cells. On the other hand, referring to FIG. 10B, in cells treated with TGF-β, the cells classified as epithelial cells decreased to 3.75%, the cells classified as intermediate cells increased to 69.1%, and the cells classified as mesenchymal cells increased to 25.4%. The threshold value set in this study was used in the study using iCCs described below and for distinguishing cells in the Examples.

### (4) Immunostaining of iCCs, IFCM measurement, and cell differentiation

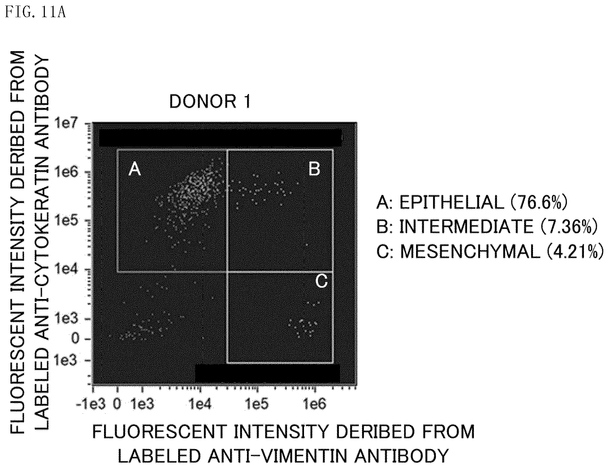

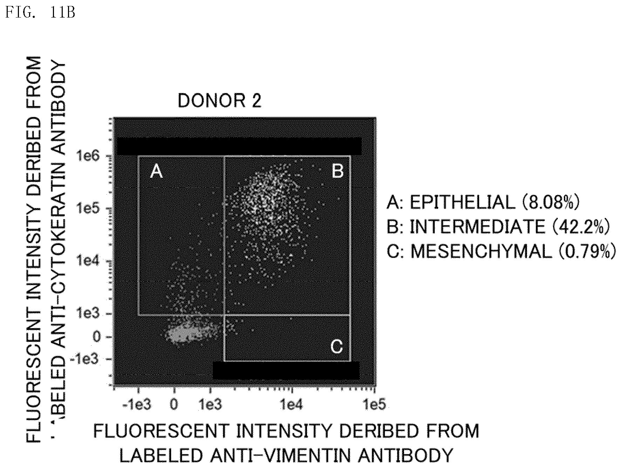

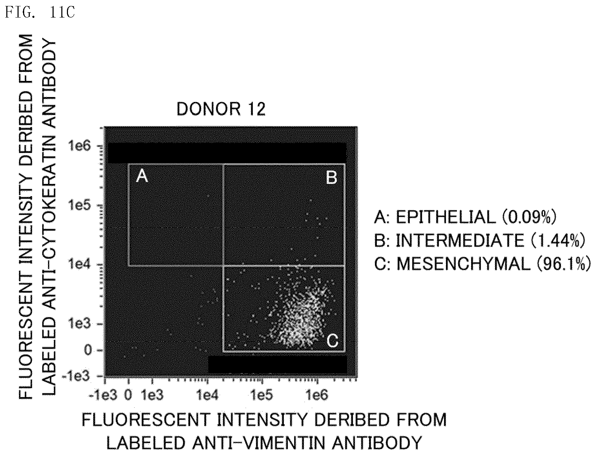

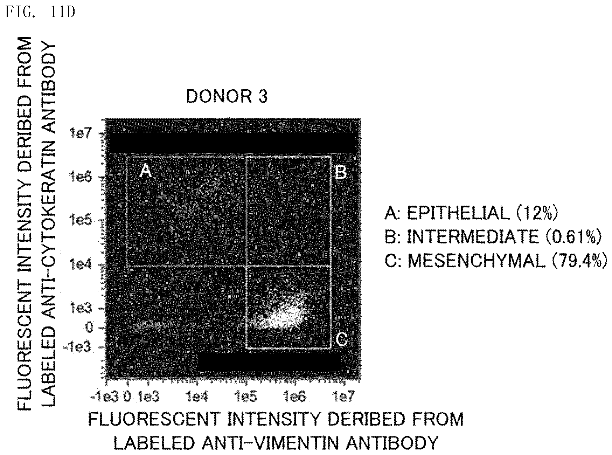

Patient-derived iCCs determined in Example 2 to express defective APC protein were immunostained using eFluor 615-labeled anti-cytokeratin antibody and PE-labeled anti-vimentin antibody in the same manner as described above in (3). Then, the samples containing the immunostained iCCs were subjected to IFCM measurement, and a scattergram was created. Based on the thresholds set in (3) above, the iCCs of each patient were classified into epithelial cells, mesenchymal cells, and intermediate cells. As an example, the scattergrams for Donor 1, Donor 2, Donor 3 and Donor 12 are shown in Figures 11A-D, respectively. FIG. 11A shows the results for Donor 1, FIG. 11B shows the results for Donor 2, FIG. 11C shows the results for Donor 12, and FIG. 11D shows the results for Donor 3. From FIGs. 11A to 11D, it was found that the iCCs from Donor 1 contained the most epithelial cells, the iCCs from Donor 2 contained the most intermediate cells, and the iCCs from Donor 12 contained the most mesenchymal cells. In addition, in iCCs from Donor 3, mesenchymal cells were the most abundant, but epithelial cells also accounted for 12%. This suggested that the iCCs from Donor 3 were a type that contained both epithelial and mesenchymal cells. Thus, it was demonstrated that iCCs expressing defective APC proteins can be distinguished as being epithelial, mesenchymal, or intermediate based on the expression of cytokeratin and vimentin.

### (5) Immunostaining, FCM measurement, and cell differentiation of HCC827 cells

As in (3) above, HCC827 cells in which EMT was induced and HCC827 cells cultured without the addition of TGF-β were prepared. These cells were treated in the same manner as in (3) above to obtain samples containing immunostained cells. The sample was measured using an FCM, FACS Verse (Becton Dickinson Japan). The measurement data was analyzed by a conventional method to obtain the intensities (fluorescence intensities) of the fluorescent signals generated by eFluor 615 and PE, respectively. A scattergram was prepared in which the vertical axis represents the fluorescence intensity derived from the eFluor 615-labeled anti-cytokeratin antibody and the horizontal axis represents the fluorescence intensity derived from the PE-labeled anti-vimentin antibody.

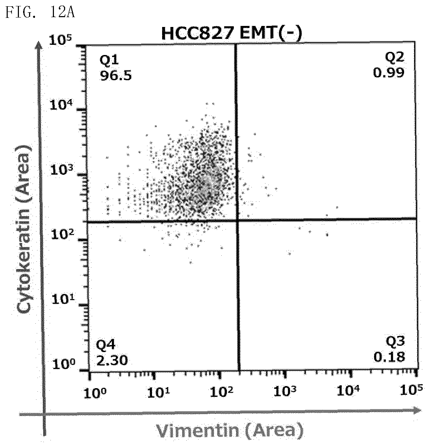

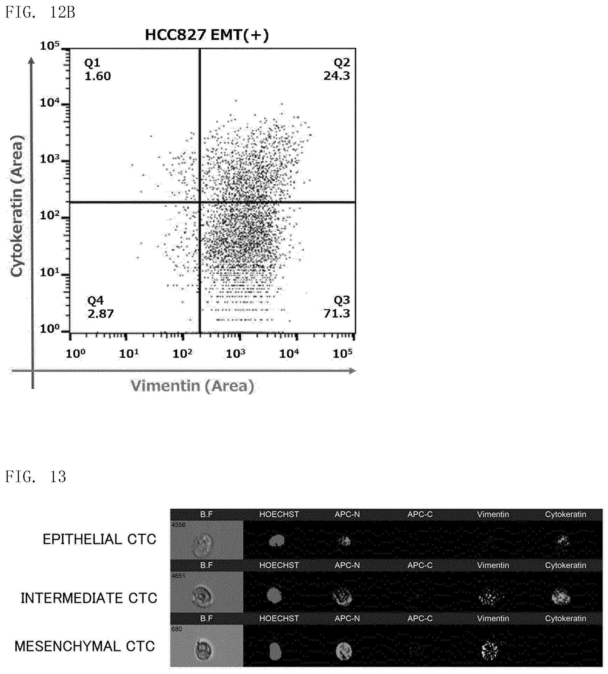

A scattergram of cells not treated with TGF-β is shown in FIG. 12A, and a scattergram of cells treated with TGF-β is shown in FIG. 12B. In the figure, "Vimentin (Area)" indicates the fluorescence intensity (area) derived from the PE-labeled anti-vimentin antibody, and "Cytokeratin (Area)" indicates the fluorescence intensity (area) derived from the eFluor 615-labeled anti-cytokeratin antibody. The scattergrams in FIGS. 12A and 12B were compared, and threshold values were set for the fluorescence intensities derived from the labeled anti-vimentin antibody and the labeled anti-vimentin antibody, respectively. In each scattergram, the vertical line represents the vimentin threshold and the horizontal line represents the cytokeratin threshold. 12A and B show that in the scattergram of cells not treated with TGF-β, cells with strong fluorescence intensity derived from the eFluor 615-labeled anti-cytokeratin antibody are detected, but cells with weak fluorescence intensity derived from the PE-labeled anti-vimentin antibody are mainly detected (upper left of Fig. 12A; 96.5% of the total), whereas in the scattergram of cells treated with TGF-β, cells with strong fluorescence intensity derived from the PE-labeled anti-vimentin antibody are mainly detected (right side of Fig. 12B; the upper right and lower right combined account for 95.6% of the total). Thus, the distribution of cells was significantly different between the scattergrams of cells not treated with TGF-β and those treated with TGF-β. These results demonstrated that FCM could also be used to determine whether iCCs were epithelial or mesenchymal, and whether the cells had undergone EMT, based on the expression of cytokeratin and vimentin.

### Example 4: Detection of circulating tumor cells expressing defective APC protein

The same immunostaining and IFCM measurement as in Example 1 were performed on CTCs derived from colon cancer patients to examine whether CTCs containing the APC protein could be detected. Furthermore, by the immunostaining and IFCM measurement performed in Example 3, it was determined whether the CTCs derived from colon cancer patients were epithelial, mesenchymal, or intermediate.

### (1) Cells and antibodies

Blood was collected from each of the colon cancer patients similar to those in Example 2. CTCs were separated and enriched from the blood of each patient using a CTC enrichment and recovery device, the ClearCell (registered trademark) FX system (Biolidics), and a separation and enrichment chip, the CTChip (registered trademark) FR1S (Biolidics). In the immunostaining described below, the same fluorescently labeled anti-APC-N and anti-APC-C antibodies as in Example 1, and the same fluorescently labeled anti-cytokeratin and anti-vimentin antibodies as in Example 3 were used.

### (2) Immunostaining and IFCM measurement

CTCs from each patient were fixed with 4% PFA solution. Fixed CTCs were washed with PBS containing 0.2% Pluronic F-127. The fixed CTCs were permeabilized with chilled methanol at a final concentration of 90%. The permeabilized cells were washed with PBS containing 0.2% Pluronic F-127. The fixed and permeabilized CTCs were added with PBS containing Alexa 488-labeled anti-APC-N antibody, Alexa 647-labeled anti-APC-C antibody, eFluor 615-labeled anti-cytokeratin antibody, PE-labeled anti-vimentin antibody, and Hoechst (registered trademark) 33342, and incubated at room temperature for 1 hour. CTCs were then washed twice with PBS containing 0.2% Pluronic F-127. This resulted in a sample containing immunostained CTCs. The sample was measured using an ImageStream MK II (Cytec Japan). The measurements involved acquiring bright field and fluorescent images of individual particles in the sample. The acquired image data was analyzed using IDEAS Application v6.2 (MERCK MILLIPORE). The fluorescent images of the particles to be analyzed were analyzed using the Intensity feature of IDEAS, and the intensities (fluorescence intensities) of the fluorescent signals arising from Alexa 488, Alexa 647, eFluor 615, and PE were obtained.

### (3) Detection of APC protein and cell differentiation

The APC-C/APC-N ratio was calculated for each patient's CTC-containing sample. CTCs containing defective APC protein were detected in samples from donors 1, 2, 3, 6, 7, 10, 11, 12, and 14. For all of these donors, the average APC-C/APC-N ratio of particles in the samples was 0.25 or less, and therefore they were considered to be "positive for defective APC protein." In the samples from the other donors, cells containing wild-type APC protein were detected, and CTCs containing defective APC protein were less than the specified amount or were not detected.

A scattergram was prepared in which the vertical axis represents the fluorescence intensity derived from the eFluor 615-labeled anti-cytokeratin antibody and the horizontal axis represents the fluorescence intensity derived from the PE-labeled anti-vimentin antibody. Based on the threshold set in Example 3, the CTCs of each patient were classified into epithelial cells, mesenchymal cells, and intermediate cells. FIG. 13 shows an example of images acquired by IFCM of epithelial CTCs, intermediate CTCs, and mesenchymal CTCs from a patient determined to be defective by IFCM measurement. Referring to FIG. 13, in epithelial CTCs, fluorescence derived from the eFluor 615-labeled anti-cytokeratin antibody was detected, but fluorescence derived from the PE-labeled anti-vimentin antibody was not detected. In mesenchymal CTCs, fluorescence derived from the PE-labeled anti-vimentin antibody was detected, but fluorescence derived from the eFluor 615-labeled anti-cytokeratin antibody was not detected. In mesenchymal CTCs, fluorescence derived from eFluor 615-labeled anti-cytokeratin antibody and PE-labeled anti-vimentin antibody was detected. In all CTCs, fluorescence derived from the Alexa 488-labeled anti-APC-N antibody was detected, but fluorescence derived from the Alexa 647-labeled anti-APC-C antibody was not detected. Therefore, it was demonstrated that CTCs expressing defective APC proteins can be detected by measuring CTCs immunostained with labeled anti-APC-N and anti-APC-C antibodies by IFCM. In addition, by performing immunostaining with anti-cytokeratin and anti-vimentin antibodies and IFCM measurement, it was shown that CTCs expressing defective APC protein can be distinguished as epithelial cells, mesenchymal cells, or intermediate cells.

### Example 5: Detection of circulating tumor cells expressing defective APC protein (2)

CTCs derived from a colon cancer patient different from the patient in Example 2 were analyzed by the immunostaining and IFCM measurement performed in Example 4, and the relationship with the stage of colon cancer was examined.

### (1) Cells and antibodies

Blood was collected from each of 79 patients with stage I to IV colon cancer. In the same manner as in Example 4, CTCs were isolated and enriched from the blood of each patient. In the immunostaining described below, the same fluorescently labeled anti-APC-N and anti-APC-C antibodies as in Example 1, and the same fluorescently labeled anti-cytokeratin and anti-vimentin antibodies as in Example 3 were used.

### (2) Immunostaining and IFCM measurement

In the same manner as in Example 4, CTCs from each patient were immunostained and IFCM measurement was performed. Based on the acquired images, for each patient, CTCs that showed detection of fluorescence derived from Alexa 488-labeled anti-APC-N antibody but not from Alexa 647-labeled anti-APC-C antibody were counted as CTCs expressing defective APC protein. A scattergram was prepared in which the vertical axis represents the fluorescence intensity derived from the eFluor 615-labeled anti-cytokeratin antibody and the horizontal axis represents the fluorescence intensity derived from the PE-labeled anti-vimentin antibody. Based on the threshold set in Example 3, the CTCs of each patient were classified into epithelial cells, mesenchymal cells, and intermediate cells. Based on the acquired images, the number of mesenchymal CTCs expressing defective APC protein was counted among the mesenchymal CTCs from each patient. The results are shown in Table 1.

**Table 1**

| STAGE | | NUMBER OF PATIENTS | NUMBER OF PATIENTS IN WHOM ONE OR MORE CTCS EXPRESSING DEFECTIVE APC PROTEIN WERE DETECTED | NUMBER OF PATIENTS IN WHOM ONE OR MORE MESENCYMAL CTCS EXPRESSING DEFECTIVE APC PROTEIN WERE DETECTED |
|---|---|---|---|---|
| STAGE | **I** | **14** | **1, (7%)** | **0,(0%)** |
| STAGE | **II** | **31** | **12, (39%)** | **3, (10%)** |
| STAGE | **III** | **28** | **11, (39%)** | **5, (18%)** |
| STAGE | **IV** | **6** | **4, (67%)** | **3, (50%)** |

As can be seen from Table 1, the proportion of patients in whom CTCs expressing defective APC protein were detected increased with progression of the disease stage. Furthermore, the more advanced the disease stage, the higher the proportion of patients in whom mesenchymal CTCs expressing defective APC protein were detected. It is generally said that the concentration of CTCs in the blood increases in correlation with the stage of cancer. Furthermore, it is said that the more malignant the cancer, the more mesenchymal CTCs are detected. It is also known that APC gene mutations that cause colorectal cancer almost always result in defective APC protein. The results shown in Table 1 were consistent with those previously reported.

### Example 6: Evaluation of storage stability of samples

From blood samples prepared from blood stored at room temperature for 24 or 48 hours, cells containing APC protein were isolated.

We investigated whether it is possible to detect cells that have undergone EMT and EMT using this method.

### (1) Detection of APC in blood stored at room temperature

Blood was collected from healthy subjects and dispensed into multiple 15 ml tubes. To each tube, HCT116 cells or DLD-1 cells suspended in PBS containing 0.2% Pluronic F-127 were added. These tubes were then allowed to stand at room temperature and collected after 24 hours and 48 hours. For comparison, a sample that was not allowed to stand at room temperature (blood immediately after addition of cells; hereafter referred to as the "sample after 0 hours") was also prepared. A hemolysis treatment was carried out by adding a hemolysis agent (G-BIOSCIENCE) in an amount four times the amount of blood to each tube and shaking and stirring at room temperature for 10 minutes. The cell pellet was collected by centrifugation and washed with PBS containing 0.2% Pluronic F-127. After washing, the cells were fixed by adding a 4% PFA solution, and the cells were washed with PBS containing 0.2% Pluronic F-127. The fixed cells were permeabilized with cold methanol at a final concentration of 90%. The cells were then washed with PBS containing 0.2% Pluronic F-127. The fixed and permeabilized cells were subjected to immunostaining of the added cells and leukocytes in the same manner as in Example 1(3). For immunostaining, the same fluorescently labeled anti-APC-N antibody and anti-APC-C antibody as in Example 1 were used. Samples containing immunostained cells were measured on an ImageStream MK II. The measurements were performed to obtain fluorescence images of individual particles in the sample. The acquired image data was analyzed using IDEAS Application v6.2. The fluorescent images of the particles to be analyzed were analyzed using the Intensity feature of IDEAS, and the intensities (fluorescence intensities) of the fluorescent signals generated by Alexa 488 and Alexa 647 were obtained.

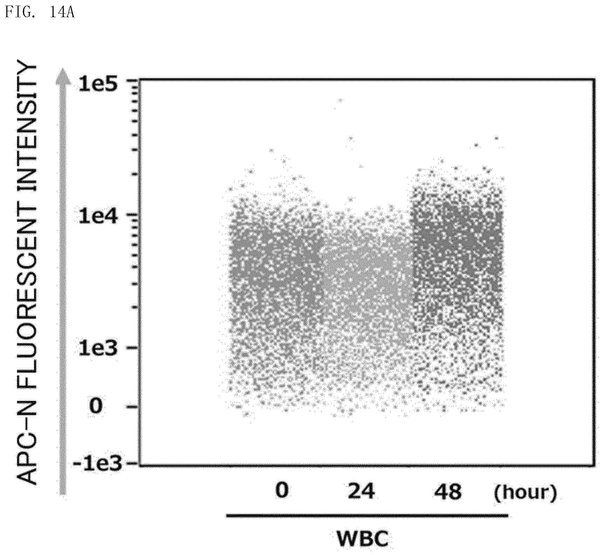

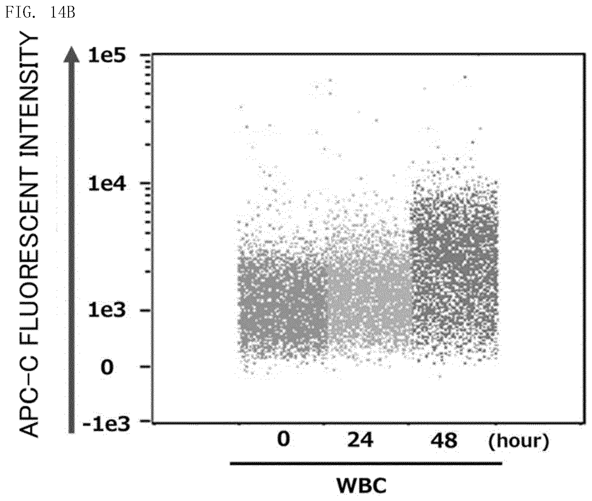

FIG. 14A shows plots of the fluorescence intensity derived from the Alexa 488-labeled anti-APC-N antibody for each sample. FIG. 14B shows plots of the fluorescence intensity derived from the Alexa 647-labeled anti-APC-C antibody for each sample. FIG. 14A shows that the intensity of fluorescence derived from the Alexa 488-labeled anti-APC-N antibody could be stably measured in all samples after 0, 24, and 48 hours. FIG. 14B shows that there was no change in the intensity of fluorescence derived from the Alexa 647-labeled anti-APC-C antibody in the samples after 0 and 24 hours, but there was a change in the fluorescence intensity in the sample after 48 hours. These results demonstrate that, in blood samples stored at room temperature for up to 24 hours after collection, cells containing defective APC protein can be detected by immunostaining and IFCM measurement using fluorescently labeled anti-APC-C and anti-APC-N antibodies.

### (2) Detection of cells that have undergone EMT using HCC827 cells

In the same manner as in Example 3(3), HCC827 cells in which EMT was induced by TGF-β and HCC827 cells cultured without the addition of TGF-β were prepared. HCC827 cells in which EMT had been induced or HCC827 cells cultured without the addition of TGF-β, suspended in PBS containing 0.2% Pluronic F-127, were added to healthy human blood that had been dispensed into multiple 15-ml tubes. These tubes were then allowed to stand at room temperature and collected after 24 hours and 48 hours. For comparison, a sample after 0 hours was also prepared. A hemolysis treatment was carried out by adding a hemolysis agent (G-BIOSCIENCE) in an amount four times the amount of blood to each tube and shaking and stirring at room temperature for 10 minutes. The cell pellet was collected by centrifugation and washed with PBS containing 0.2% Pluronic F-127. After washing, the cells were fixed by adding a 4% PFA solution, and the cells were washed with PBS containing 0.2% Pluronic F-127. The fixed cells were permeabilized with cold methanol at a final concentration of 90%. The cells were then washed with PBS containing 0.2% Pluronic F-127. The cells that had been added to the fixed and permeabilized cells were immunostained in the same manner as in Example 3(3) above. For immunostaining, the same eFluor 615-labeled anti-cytokeratin antibody and PE-labeled anti-vimentin antibody as in Example 1 were used. Samples containing immunostained cells were measured on an ImageStream MK II. The measurements were performed to obtain fluorescence images of individual particles in the sample. The acquired image data was analyzed using IDEAS Application v6.2. The fluorescent images of the particles to be analyzed were analyzed using the Intensity feature of IDEAS, and the intensities (fluorescence intensities) of the fluorescent signals generated by eFluor 615 and PE were obtained.

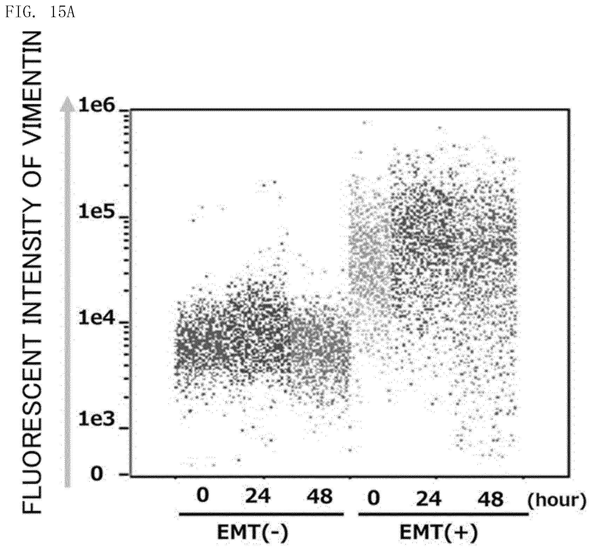

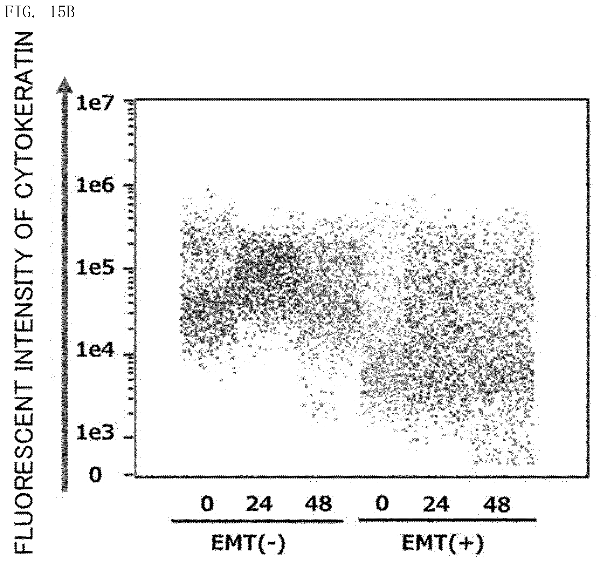

FIG. 15A shows the results of plotting the fluorescence intensity derived from the PE-labeled anti-vimentin antibody for each sample. Furthermore, the fluorescence intensity derived from the eFluor 615-labeled anti-cytokeratin antibody for each sample is plotted in FIG. 15B. In the figure, "EMT(-)" indicates HCC827 cells cultured without the addition of TGF-β, and "EMT(+)" indicates HCC827 cells in which EMT was induced. As shown in FIG. 15A, in all samples after 0, 24 and 48 hours, the fluorescence intensity derived from the PE-labeled anti-vimentin antibody was higher in the EMT(+) samples than in the EMT(-) samples. This shows that HCC827 cells in which EMT was induced could be distinguished from HCC827 cells cultured without the addition of TGF-β. FIG. 15B shows that the fluorescence intensity derived from the eFluor 615-labeled anti-cytokeratin antibody was stably measured in all samples after 0 hours, 24 hours, and 48 hours. These results demonstrate that EMT cells can be detected by immunostaining with fluorescently labeled anti-cytokeratin and anti-vimentin antibodies and IFCM measurement, even in blood samples stored at room temperature for 48 hours after collection.

### [Explanation of symbols]

10, 20, 30, 40: Reagent kit
11: Container
12, 23, 34, 45: Packing box
13, 24, 35, 46: Package insert
21, 31, 41: First container
22, 32, 42: Second container
33, 43: Third container
44: Fourth Container

## Claims

1. A method for detecting a circulating tumor cell in a sample, comprising the steps of:
contacting the circulating tumor cell in the sample with a first labeled antibody comprising a first labeling substance and a second labeled antibody comprising a second labeling substance that is different from the first labeling substance; and
detecting a circulating tumor cell containing a defective APC protein to which the first labeled antibody is bound and to which the second labeled antibody is not bound,
wherein the first labeled antibody comprises an antibody capable of binding to a wild-type APC protein and a defective APC protein, and the second labeled antibody comprises an antibody capable of binding to the wild-type APC protein and not to the defective APC protein.

2. The method of claim 1, further comprising the step of detecting cells containing a wild-type APC protein bound to the first labeled antibody and the second labeled antibody.

3. The detection method according to claim 1 or 2, wherein the detecting step comprises:
detecting a first signal derived from the first labeling substance and a second signal derived from the second labeling substance; and
detecting a circulating tumor cell containing the defective APC protein based on the first signal and the second signal.

4. The detection method according to any of claims 1 to 3, wherein the detecting step comprises:
detecting a first signal derived from the first labeling substance and a second signal derived from the second labeling substance; and
detecting a cell in which a ratio of the second signal to the first signal is less than a threshold value, or a cell in which a ratio of the first signal to the second signal is equal to or greater than a threshold value, as a circulating tumor cell comprising the defective APC protein.

5. The detection method according to any of claims 1 to 4, wherein the detecting step comprises:
acquiring an image of a cell in the sample, and detecting a first signal derived from the first labeling substance and a second signal derived from the second labeling substance in the image of the cell; and
detecting a cell comprising the first signal and substantially not comprising the second signal as a circulating tumor cell containing the defective APC protein.

6. The detection method according to claim 5, wherein the first labeling substance is a fluorescent substance, the second labeling substance is a fluorescent substance, the first signal is a fluorescent signal, the second signal is a fluorescent signal, and the image is a fluorescent image.

7. The detection method according to any of claims 1 to 6, wherein the defective APC protein is a protein in which the C-terminal region of the wild-type APC protein is deleted.

8. The detection method according to any of claims 1 to 7, wherein the first labeling substance and the second labeling substance are fluorescent substances having fluorescence emission maxima in different wavelength ranges.

9. The detection method according to any of claims 1 to 8, further comprising the steps of:
labeling a mesenchymal marker protein of the circulating tumor cell; and
detecting a cell to which the first labeled antibody binds, in which the mesenchymal marker protein is labeled, and to which the second labeled antibody does not bind, as a mesenchymal circulating tumor cell that comprises the defective APC protein.

10. The detection method according to claim 9, wherein the mesenchymal marker protein is at least one selected from the group consisting of vimentin, N-cadherin, OB-cadherin, fibronectin, integrin A5bl, Snail, Slug, ETS1, *α* -SMA, Twist, FAP, FSP-1, SIP1, Goosecoid, LEF-1, and FOXC2.

11. The detection method according to any of claims 1 to 10, further comprising the steps of:
labeling an epithelial marker protein of the circulating tumor cell; and
detecting a cell to which the first labeled antibody and the second labeled antibody bind and in which the epithelial marker protein is labeled, as an epithelial cell comprising the wild-type APC protein.

12. The detection method according to claim 11, wherein the epithelial marker protein is at least one selected from the group consisting of cytokeratin, EpCAM, E-cadherin, ZO-1, laminin-1, entactin, MUC1, desmoplakin, and α1 collagen.

13. The detection method according to any of claims 1 to 12, wherein the first labeled antibody binds to a site present in both the wild-type APC protein and the defective APC protein, and the second labeled antibody binds to a site present in the wild-type APC protein but not in the defective APC protein.

14. A method for detecting circulating tumor cell in a sample which comprises a defective APC protein to which a first labeled antibody is bound and a second labeled antibody is not bound,
wherein the circulating tumor cell is prepared by contacting the circulating tumor cell in the sample with the first labeled antibody and the second labeled antibody,
the first labeled antibody comprising a first labeling substance and an antibody capable of binding to a wild-type APC protein and a defective APC protein, and the second labeled antibody comprising a second labeling substance different from the first labeling substance and an antibody capable of binding to the wild-type APC protein and not to the defective APC protein.

15. The method according to any one of claims 1 to 14, wherein the detection of circulating tumor cells is carried out with a fluorescent microscope or a flow cytometer.
